(19) Europäisches Patentamt
European Patent Office
Office européen des brevets

(11) **EP 4 755 904 A2**

(12) **EUROPEAN PATENT APPLICATION**

(43) Date of publication:
**10.06.2026   Bulletin 2026/24**

(21) Application number: **26160510.9**

(22) Date of filing: **24.03.2023**

(51) International Patent Classification (IPC):
**C07K 14/46** [(2006.01)]

(52) Cooperative Patent Classification (CPC):
(C-Sets available)
**C07K 14/46; A01N 37/46; A01P 11/00**      (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:   **24.03.2022   GB 202204128**

(62) Document number(s) of the earlier application(s) in accordance with Art. 76 EPC:
**23716649.1 / 4 499 665**

(71) Applicant: **Pelgen Limited**
**Alton Hampshire GU34 2QR (GB)**

(72) Inventors:
• **TRIM, Steven**
  **Sandwich CT13 9FE (GB)**
• **MCCULLOUGH, Danielle**
  **Sandwich CT13 9FE (GB)**

(74) Representative: **Boxall, Sarah Jane**
**Boxall IPM Ltd**
**9 King Street**
**Sandwich Kent CT13 9BT (GB)**

Remarks:
•The complete document including Reference Table(s) and the Sequence Listing(s) can be downloaded from the EPO website
•This application was filed on 24-02-2026 as a divisional application to the application mentioned under INID code 62.
•Claims filed after the date of filing of the application/ after the date of receipt of the divisional application (Rule 68(4) EPC).

(54)    **RODENTICIDE**

(57)    Described are novel synthetic peptide sequences based on Elapid snake venom that are suitable for use as a cardiotoxic rodenticide.

Figure 8B

(52) Cooperative Patent Classification (CPC): (Cont.)

C-Sets
**A01N 37/46, A01N 25/004**

**Description**

The present invention relates to synthetic peptides, a rodenticide based on Elapid snake venom, in particular cardiotoxins derived from snake venom, and the use of a cardiotoxin as a rodenticide.

Rats and mice are inevitably associated with human habitation, and this can have largely negative outcomes as rodents notoriously consume produce and stored resources. They can cause destruction of crops, cereals and grains, accounting for nearly 10% of the grain crops in the world, with variation depending on country (Buckle and Smith, (2015). Rodent Pests and their control. 2nd Edition. Wallingford Oxfordshire. 330-345). They also transmit zoonotic diseases to humans and domestic animals and have been known to gnaw and burrow causing substantial damage to buildings and power supplies (Van den Brink, et al, (2018) In Anticoagulant Rodenticides and Wildlife; Springer: Cham, Switzerland, pp. 1-9). Rodent control is thus crucial and routine worldwide, particularly in agricultural settings and in and around buildings. The approach to managing and controlling rodent populations takes several forms: improving sanitation and rodent-proofing areas, capturing and moving rodents elsewhere and lethal control tools such as traps or rodenticides. A combination of efficacy, ease of use, cost-effectiveness and the fact that they are relatively safe for the operator makes the use of anticoagulants (ARs) the most appealing approach to rodent control (Jakob and Buckle, (2018). Emerging Topics in Ecotoxicology (Principles, Approaches and Perspectives), vol 5. Springer, Cham.). The sophisticated social organisation of rodent populations and the neophobic behaviour of rats makes the action of toxicity with immediate effect ineffective (Modlinska and Stryjek, (2016) PLoS ONE 11(6)). Anticoagulant rodenticides address this issue as they have delayed action and mortality typically occurs several days after consumption. Prolonged or repeated exposure to ARs leads to rodent death by haemorrhage within 3-7 days.

Anticoagulant rodenticides (ARs) work by inhibiting vitamin K epoxide reductase (VKOR) and hence deplete vitamin-K dependent clotting factors (Ng WY, et al (2018) Journal of Medical Toxicology; 14(3):218-228). ARs inhibit vitamin K(1)-2.3 epoxide reductase thus inhibiting the synthesis of vitamin K and subsequently clotting factors II, VII, IX and X (Hadler and Buckle, (1992) Proceedings of the Vert Pest Conference, 15:149-155). The increased potency and duration of action of long-acting ARs is attributed to their greater affinity for vitamin K(1)-2,3-epoxide reductase and their ability to disrupt the vitamin K(1)-epoxide cycle at more than one point (Watt, et al, (2005) Toxicology Reviews. 24(4):259-69). They also have long biological half-lives due to high lipid solubility and can be reabsorbed into circulation via enterohepatic recycling.

Warfarin (4-hydroxy-3-(3-oxo-1-phenylbutyl)chromen-2-one) was one of the first anticoagulant rodenticides (ARs) brought to market but resistance has developed in rodents.

Coumatetralyl (4-hydroxy-3-(1,2,3,4-tetrahydronaphthalen-1-yl) chromen-2-one), despite its reported low toxicity, is slightly more effective than warfarin due to its higher palatability. The compound was introduced on the emergence of warfarin-resistant rat populations and was successful for many years before resistance was reported in the United Kingdom and Denmark in the late 1960s.

Second generation anticoagulant rodenticides (SGARs) were developed in the 1970s to overcome developing resistance against first-generation anticoagulant rodenticides (FGARs) in rats. They are more acutely toxic than first generation ARs and their enhanced potency is related to their greater affinity for vitamin K epoxide reductase (VKOR). Bromadiolone (3-[3-Bromo[1,1'-biphenyl-4-yl]-3-hydroxy-1-phenylpropyl]-4-hydroxy-2H-1-benzopyran-2-one) and Difenacoum (3-[3-p-diphenyl-1,2,3,4-hydronaphth-1-yl]-4-hydroxycoumarin) were the first compounds introduced to the market and the three most potent compounds are Brodifacoum (3-[3-(4'-bromobiphenyl-4-yl)-1,2,3,4-tetrahydro naphth-1-yl]-4-hydroxycoumarin), Flocoumafen (4-hydroxy-3-[l,2,3,4-tetrahydro-3-[4- (4-trifluormethylbenzyloxy)phenyl-l-napthylcoumarin) and Difethialone (3-[3-[4-(4-bromophenyl)phenyl]-1,2,3,4-tetrahydronaphthalen-1-yl]-2-hydroxythiochromen-4-one).

In populations of resistant rats, VKOR is slightly changed thus preventing correct binding with the rodenticide which then fails to work (Thijssen, (1995) Pesticide Sciences 43;73-78). The most prolific resistance mechanism is a result of single-nucleotide polymorphisms in the VKORC1 gene (Rost, et al (2004) Nature. 427(6974):537-41; Li, et al (2004) Nature 427:541-544). The VKOR1 gene codes for the VKOR1C protein that contains 163 amino acids and specific amino acid substitutions in VKORC1 have been shown to present resistance to ARs. It has been suggested that at least seven independent polymorphisms occur in the VKORC1 gene in Norway rats that provide the genetic basis for anticoagulant rodenticides resistance (Mooney, et al (2018) Nature. 8:4543). It has been observed that resistance expanded geographically and progressed from warfarin and FGARs, to the more potent active substances SGARs such as bromadiolone and difenacoum. Resistance to different ARs is called cross-resistance and evolves from FGARs to SGARs and as a consequence resistance to SGARs will always be accompanied by a resistance to FGARs (Mooney, et al (2018) supra).

The use of FGARs is now largely limited due to this genetic resistance phenomenon; extensive cases of resistance in brown rats has been reported in the United Kingdom, Europe, the United States, Canada and Australia since 1958 (Buckle, et al (1994) Proceedings of the Sixteenth Vertebrate Pest Conference. 7). Mutations in VKORC1 have been shown to cause two different hereditary phenotypes; defective blood coagulation owing to vitamin K-dependent coagulation factor deficiency type 2 (VKCFD2) and warfarin resistance (OMIM) (Rost, et al (2009) BMC genetics).

Brodifacoum, Flocoumafen and Difethialone are currently the only active compounds that have demonstrated efficacy in all resistant strains of both rats and mice.

ARs on the market are formulated into palatable baits for oral administration to targeted rodent pests and are typically based on cereal grain made into blocks or pellets, with the addition of binding agents, flavouring, and colouring. Wax is added to some products to make it longer lasting and add an element of weather proofing (Horak, et al (2018) In Anticoagulant Rodenticides and Wildlife; Springer: Cham, Switzerland, pp. 87-108). Bait formulations rely on voluntary ingestion of adequate quantities for efficacy against targeted pests. As death from AR poisoning occurs days after ingestion, it is quite typical that rodents continue to eat baits after ingesting a lethal dose, thus increasing the concentration of anticoagulant rodenticide in their tissue. Unfortunately, this also presents the possibility for unwanted primary exposure of other animals that can access the bait, and secondary oral exposure can also occur via ingestion of animal tissues that contain AR residues. This is a hazard for species that prey on rodents or scavenge the carcasses of poisoned animals (Horak, *et al* (2018) *supra).*

The unintentional exposure of non-target wildlife and domestic pets, either through primary or secondary poisoning, and reports of AR residues in predatory birds and non-target species are widespread globally. Primary exposure is a high risk for omnivorous or herbivorous animals as rodenticide bait formulations are cereal-based and secondary poisoning is characteristically seen in scavenging or predatory animals, as a result of consuming tissues from animals that carry residual concentrations of ARs (Fisher, et al (2019) Animals (Basel). 9(11):919). The higher toxicity and metabolic persistence of SGARs impart a higher risk of secondary mortality to non-target species (Erikson and Urban, (2004) Potential risks of Nine Rodenticides to Birds and Nontarget Mammals: A Comparative Approach; US Environmental Protection Agency: Washington, DC, USA, 2004).

The risks to non-target birds and other wildlife are influenced by species specific susceptibility and the toxicokinetics of the compounds used: for example, brodifacoum is highly toxic to birds and mammals (Eason, et al (2002) Ecotoxicology. 1:35-48). Second generation anticoagulant rodenticides (SGARs) such as brodifacoum, bromadiolone and difenacoum are extremely persistent in organs and tissue such as the liver, kidney and pancreas, for at least 6 months, and are toxic with a lethal dose delivered through a single feeding (Environmental Protection Agency 2004 (www.fluoridealert.org/pesticides/EPA-HQ-OPP-2006-0955-0005.pdf); Erickson and Urban, (2004) *supra).*

Poisoning from rodenticides is one of the most common types of toxicities to dogs managed by Pet Poison Helpline (Pet poison helpline website, accessed 2021) and is reported as amongst the common causes of poisoning in dogs worldwide (Seljetun, et al (2020) Acta Veterinaria Scandinavica 62:30). A study conducted in 2020 determined that brodifacoum and difenacoum can be present in dog faeces after a single ingestion for more than 700 days and 650 days respectively (Seljetun, *et al* (2020) *supra).* In a litter of puppies born from an exposed dog, low faecal concentrations were detected for 28 days after parturition. Dogs allowed to roam may be more likely to encounter rodent baits, that are perhaps improperly placed, or baits that have been dragged into areas by rodents (Merola, (2002) Vet Med 97:716-722).

Human exposure to anticoagulants through rodenticide bait can occur (Tran and King, (2016) Journal of Epidemiological Research. 2(2); Watt, *et al* (2005) *supra)* and is typically oral; accidental or deliberate. Most cases of AR exposure in humans involve young children and, as a consequence, the amounts ingested are small. Intentional ingestion of large quantities of ARs may cause anticoagulation for several weeks or months (Watt, *et al* (2005) *supra).* Substantial ingestion will cause epistaxis, widespread bruising, haematomas and gastrointestinal bleeding, and severe blood loss may result in hypovolaemic shock and death.

Due to the emergence of resistant strains of rodents and the environmental and non-target species risk of the use of secondary generation anticoagulant rodenticides, development of an alternative rodenticide is becoming more pressing. An alternative that reduces the risk to non-target species would be beneficial for the control of pest rodents, and a reduction of persistence in the environment would be advantageous. It is clear that rodent species are becoming more resistant to SGARs as well as FGARs, with brodifacoum being the only exception currently. Brodifacoum is extremely stable in the environment and has been shown to demonstrate consistent potency for up to 30 days and only requires one dose to induce mortality. It has become more common to use this more potent, and toxic rodenticide which is typically detrimental to the environment and other species. An alternative that can provide the opportunity to target primarily the intended rodents, minimise the risk of primary and secondary exposure to non-target species such as pets and predatory birds and to have a short half-life to reduce the detrimental effect on the environment would be the most appealing approach to rodent control.

Accordingly, it is against this background that the present invention has been devised.

In one aspect, the present invention resides a rodenticide comprising at least one compound that is cardiotoxic to rodents.

Preferably, at least one compound is selectively or specifically cardiotoxic. Expressed in another way, the at least one compound has no, essentially no, or no significant or detectable toxicity towards other tissues, such as neurotoxicity or haemolysis activity.

In one embodiment, the compound has an excitatory or tachycardic, rather than inhibitory, cardiotoxic effect. Expressed in another way, the compound causes a change in heart beat rate. Preferably, the compound increases heart rate and causes tachycardia. For the avoidance of doubt, an inhibitory cardiotoxin slows down and/or stops heart beat rate, whereas an excitatory cardiotoxin speeds up heart beat rate, such effect being termed tachycardia. Slowing down beat rate causes a reduction in oxygen supply to essential organs (like the brain) and so is likely to cause death more swiftly. Tachycardic cardiotoxins increase heart rate, putting additional strain on the heart over time and are more likely to result in cardiac failure through heart attack. Ultimately, both result in the same outcome, which is expiration of the animal, but through differing mechanism.

In a particular embodiment, the compound is an isolated amino acid sequence obtained or derived from snake venom

An advantage of using snake venom as a rodenticide is the environmental impact. Snake venom is broken down by digestion and so will not linger in a food chain. Additionally, because venom is a biological composition, it will be rapidly broken down in the environment and not build up in soil, for example. Therefore, a rodenticide as contemplated by the present invention provides significant advantages over existing synthetic chemicals.

In one embodiment, the snake venom is obtained or derived from a species from the family of *Elapidae.*

*Elapidae* (commonly known as elapids) is a family of venomous snakes characterised by their permanently erect fangs at the front of the mouth. Most species have neurotoxins in their venom, for immobilising prey and defence, which is channelled by their hollow fangs, while some may contain other toxic components in various proportions. The main group of toxins are Phospholipase A2 (PLA2) and Three finger toxins (3FTx). Other toxic components in some species comprise cardiotoxins and cytotoxins, which cause heart dysfunctions and cellular damage, respectively.

Preferably, the snake venom is derived or obtained from a species of cobra. Cobra is the common name of various elapid snakes, most of which belong to the genus *Naja.* All members of the group are venomous to varying extents, and some are considered among the world's most dangerous snakes, based upon their murine $LD_{50}$ values and patient lethality.

In one embodiment, the amino acid sequence is cardiotoxic, preferably selectively cardiotoxic as defined and described hereinabove. In contrast, the neurotoxic effects of snake venom are recognised to be effected via nicotinic acetylcholine receptors at neuromuscular junctions, the effect of which is respiratory arrest and death.

It is to be understood that isolated whole snake venom may be used. Such venom may be isolated from snakes by typical methods such as milking. Alternatively, the venom may be synthetic. The use of complete or whole venom minimises any additional processing and formulation required because the proteins are already in their native, active forms. However, a drawback is the additional presence of other proteins, including neurotoxins.

Another consideration is that the cytotoxicity of snake venom affects the mucus membrane in rodents which will then make the rodents averse to the taste.

A yet further consideration is the speed of cidal activity. Snake venom can kill a mouse in under a minute, as seen during predation. However, the sight of one or more dead rodents next to or near baited food will deter other rodents from eating the food and hence the poison. So, a delay in fatal action is desirable.

Accordingly, and as a result, another aspect of the present invention encompasses a synthetic or artificial peptide sequence comprising a sequence based on or derived from an Elapid or cobra three-finger toxin (3FTx).

3FTx is a protein superfamily of small toxin proteins found in the venom of snakes. The second-largest class of 3FTx proteins cause toxicity in cardiac myocytes and can cause increased heart rate and eventually cardiac arrest. Short cardiotoxins (CTXs) are single-chain polypeptides of 59-61 amino acids arranged in a three-finger fold made of anti-parallel β-strands, fortified by 4 disulphide bonds and numerous hydrogen bonds. Long three finger cytotoxins are approximately 81 amino acids. CTXs are basic proteins with hydrophobic three-finger loops, which extremities are flanked by cationic residues (mainly lysine and arginine). The hydrophobic termini impart the amphilicity of CTXs, mediating their binding and insertion into anionic phospholipid membranes that leads to deleterious cellular events such as pore formation and lysis, increased intracellular $Ca^{2+}$ ion influx and membrane depolarisation and, importantly, internalisation of the toxin leading to mitochondrial and lysosomal damages, and the disturbances of cellular cascades resulting in cell death.

It will be appreciated that the whole cytotoxin could be used in accordance with aspects of the present invention. However, it is generally more economical to use only a part. In addition, a shorter peptide sequence requires little or no folding or re-folding, or the insurance of correct folding, after production. Accordingly, it is advantageous to use a peptide that only has a simple secondary structure. In addition, the secondary structure of a peptide may be engineered to enhance certain properties such as stability, thermal stability, bioavailability and species selectivity.

In a particular embodiment, the sequence is based on or derived from Loop I of Elapid or cobra three-finger toxin. The cytotoxic effects of 3FTx are believed to reside in Loop I sequence, together with some amino acid residues on the base of Loop II (Menez et al (1990) Biochimie 72:575-588).

In one embodiment, the sequence is based on or derived from amino acid residues 1 to 30 of cobra three-finger toxin. Ideally, the sequence has between 10 and 15 amino acid residues, for example 13 amino acid residues. It will be appreciated that the ranges specified herein encompass all discrete numbers between the lower and upper numbers, such as 6, 11, 14, 21, 25 and 27 amino acids.

In one embodiment, the sequence has the following sequence:

LKC(H/N)KL(V/I)PX(V/A)(W/Y)KT [SEQ ID NO:1]

For example, the sequence may have the following sequences and variants thereof:

LKCHKLVPPVWKT [SEQ ID NO:2]
LKCNKLIPLAYKT [SEQ ID NO:3]
LKCHKLIPIAWKTK [SEQ ID NO:4]

It is helpful if the peptide includes one or more hydrophobic amino acids at one or both ends of the sequence to help with membrane penetration. Additionally or alternatively, the sequence may include one or more amino acids to enhance solubility in water. It will be appreciated that the sequence may also be engineered to include amino acids that enhance solubility in hydrophobic materials such as wax.

Further, the sequence may include one or more synthetic amino acids, it may be PEGylated and/or include a detectable tag such as a UV-fluorescent tag. Tags detectable under UV light are of particular interest because rodent urine is visible under UV light. The inclusion of a UV-fluorescent tag may then be used to assess and/or monitor a decline in the rodent population.

It will be appreciated that, in the context, it is ideal if the sequence is cardiotoxic, preferably selectively cardiotoxic, causing a change in heart beat rate as described herein above. Preferably, the change in beat rate is an increase, i.e. causing tachycardia.

The present invention also encompasses a rodenticide formulation comprising a synthetic peptide sequence as described hereinabove, formulated together with a carrier. The formulation may be a paste, solid, powder, gel or liquid and may additionally include one or more of: wax (petroleum or plant derived), cereal, grains, nuts, fruit, colourants, flavouring, herbs, spices, essential oils, oil, fat or other material palatable to rodents.

The present invention will now be described in further detail with reference to the following non-limiting examples and figures in which:

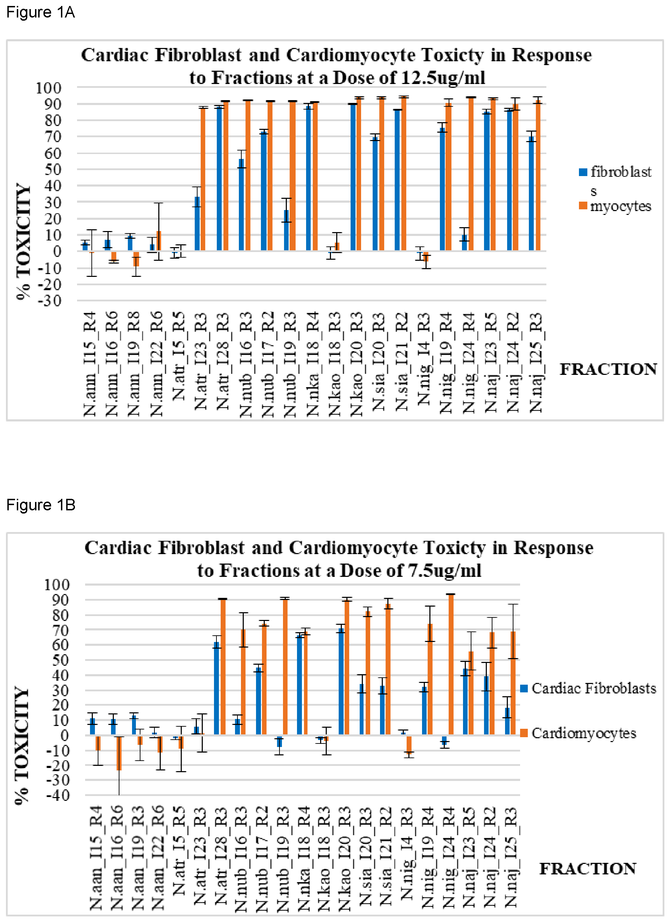

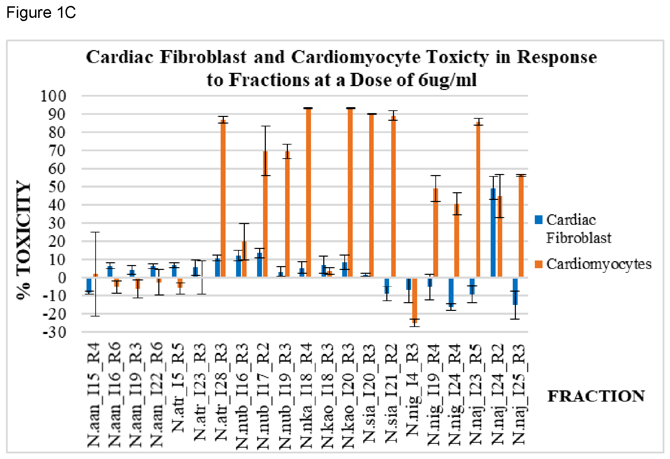

**Figure 1:** Rodent cardiomyocyte and cardiac fibroblast toxicity data. Figure 1 shows the toxicity observed in neonatal rat cardiomyocytes (orange) and cardiac fibroblasts (blue) in response to treatment with a panel of purified cobra peptides at doses of 12.5ug/ml **(Figure 1A)**, 7.5ug/ml **(Figure 1B)** and 6ug/ml **(Figure 1C)**.

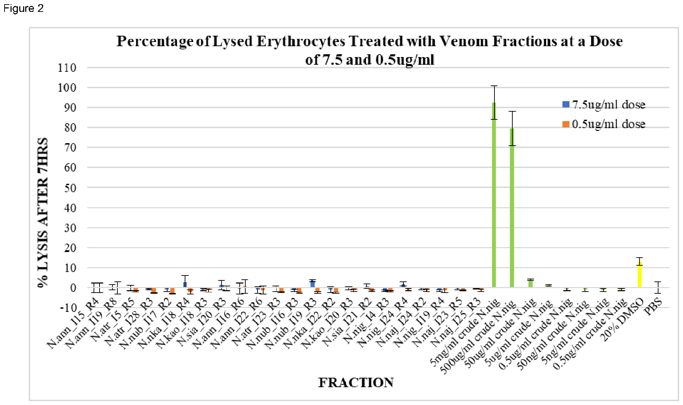

**Figure 2:** Rodent erythrocyte toxicity data. Figure 2 shows the toxicity of purified cobra fractions against isolated rat erythrocytes (red blood cells).

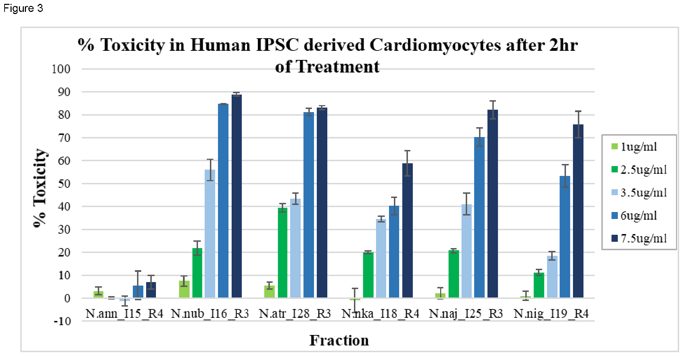

**Figure 3:** Human IPSC-derived cardiomyocyte toxicity of six lead fractions. Figure 3 shows the % toxicity to human IPSC-derived cardiomyocytes following treatment with six selected purified fractions, at doses of 1, 2.5, 3.5, 6 and 7.5$\mu$g/ml.

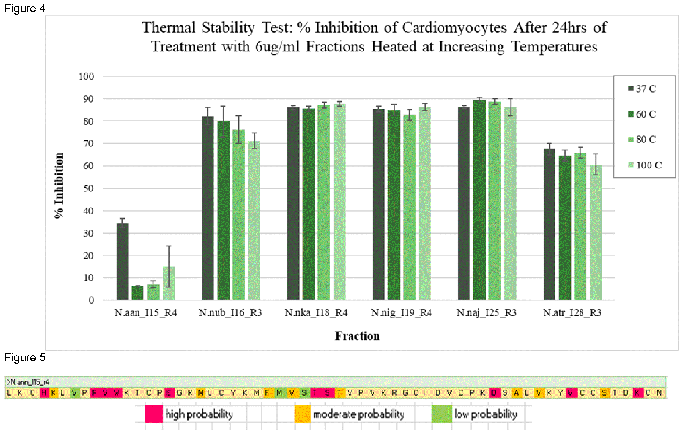

**Figure 4:** Thermal stability assessment of the six selected lead fractions. Figure 4 shows the thermal stability profiles of the six selected purified venom fractions. Fractions were heated at 37°C, 60°C, 80°C and 100°C, before being used to dose rat cardiomyocytes at 6ug/ml for 24h. The amount of cardiomyocyte toxicity was assessed at each temperature and used to determine whether there was a loss in activity due to a lack of peptide thermal stability.

**Figure 5:** SAR analysis. Figure 5 is a schematic showing amino acid residues highlighted by SAR as potentially critical to cardiomyocyte toxicity.

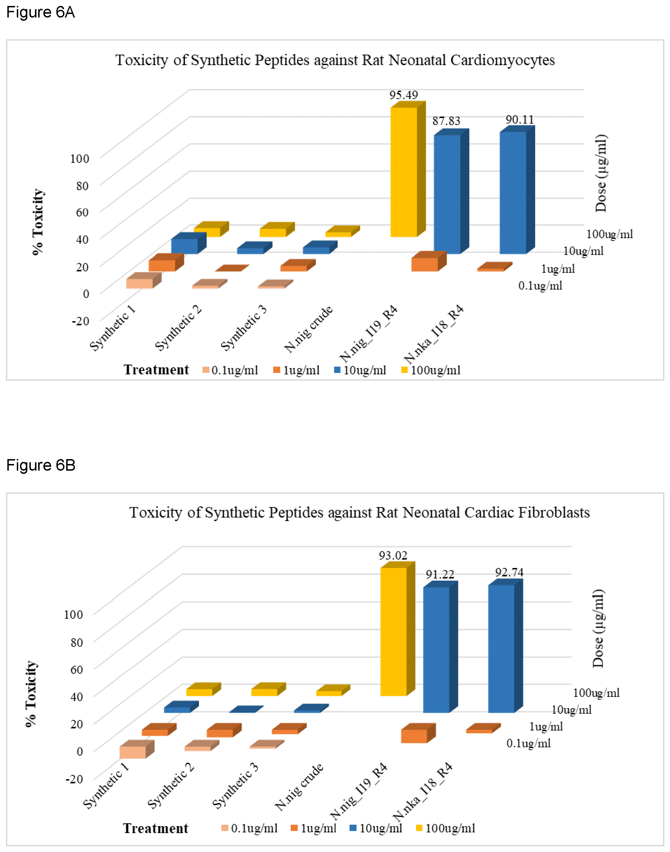

**Figure 6:** Assessing the toxicity of synthetic peptides against neonatal rat cardiomyocytes and cardiac fibroblasts using resazurin viability assays. Figure 6 shows the toxicity of the three synthetically designed short peptides against neonatal rat cardiomyocytes **(Figure 6A)** and cardiac fibroblasts **(Figure 6B)**.

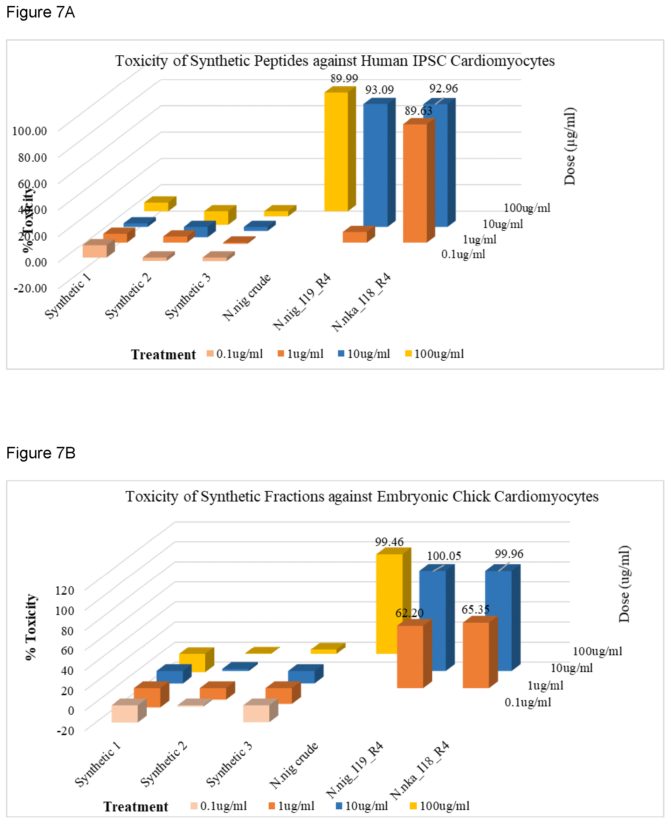

**Figure 7:** Toxicity of synthetic peptides against human IPSC-derived and Embryonic Chick Cardiomyocytes. **Figure 7A** shows the toxicity of the three synthetically designed short peptides against human IPSC-derived; **Figure 7B** shows the toxicity of the three synthetically designed short peptides against embryonic chick cardiomyocytes.

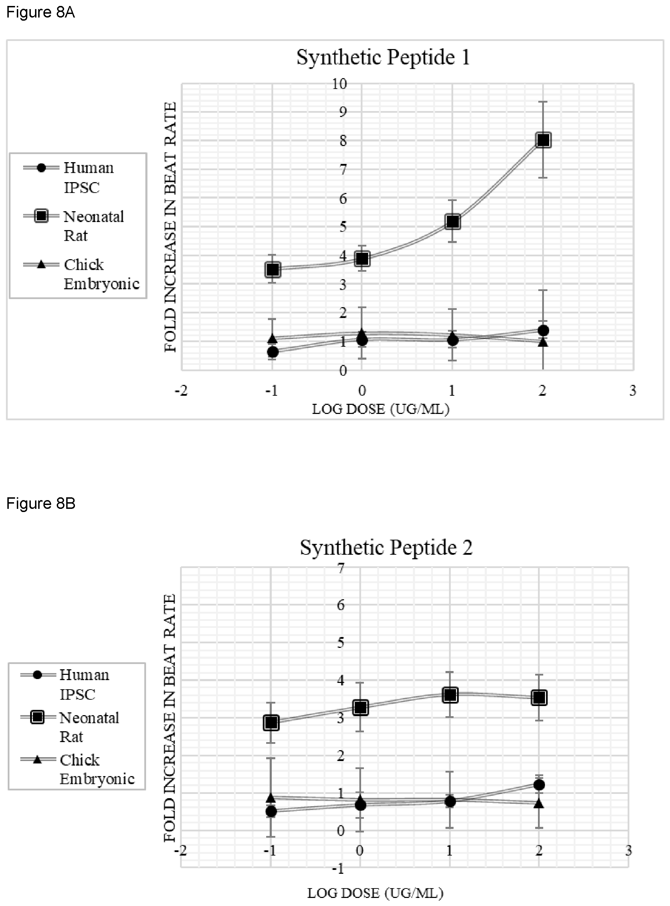

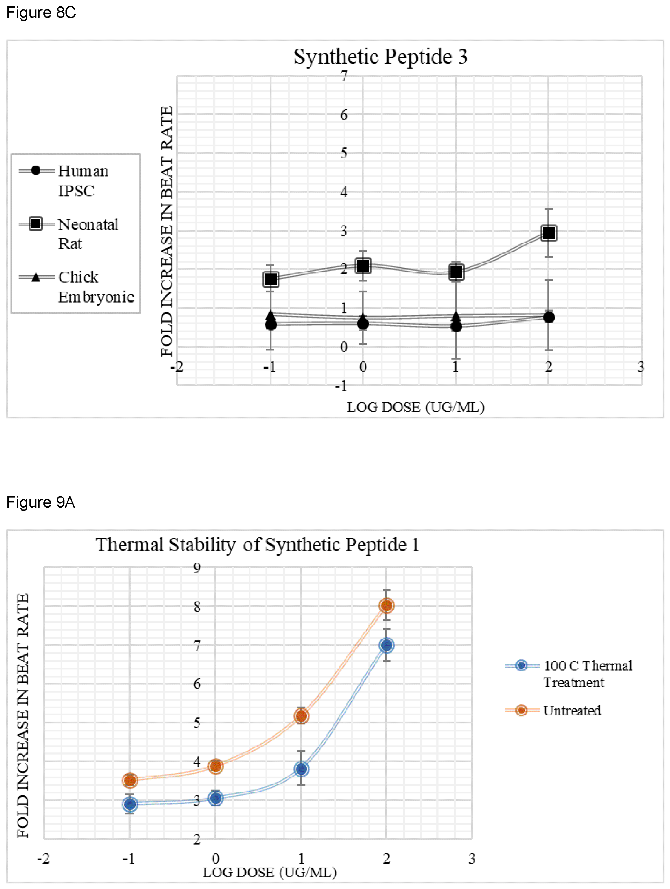

**Figure 8:** Fold changes in beat rate in neonatal rat and human IPSC-derived cardiomyocytes in response to treatment with synthetic peptides. Figure 8 shows the fold increases in beat rate in rat neonatal cardiomyocytes (orange) and human IPSC-derived cardiomyocytes (blue) in response to treatment with synthetic peptide 1 **(Figure 8A),** synthetic peptide 2 **(Figure 8B)** and synthetic peptide 3 **(Figure 8C)**.

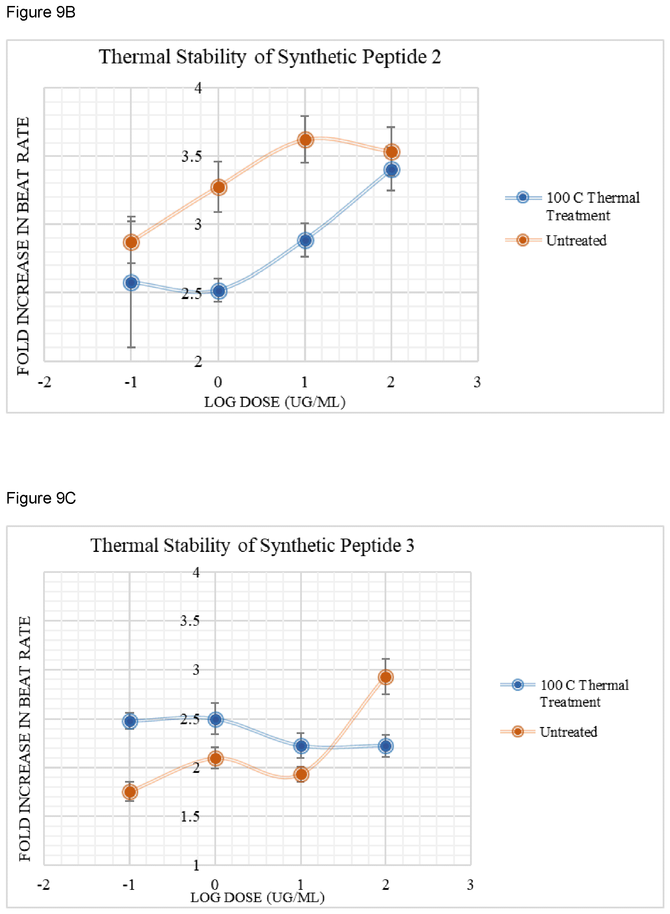

**Figure 9:** Thermal stability of synthetic peptides, assessed by fold-increases in neonatal rat cardiomyocyte beat rates. Figure 9 shows the thermal stability of synthetic peptides 1 **(Figure 9A),** 2 **(Figure 9B)** and 3 **(Figure 9C)** using fold-change in beat rate to detect loss of activity. All three synthetic fractions showed a slight loss in activity in response to heating to 100°C (blue) when compared to the same beat rates obtained following treatment with unheated fractions (orange).

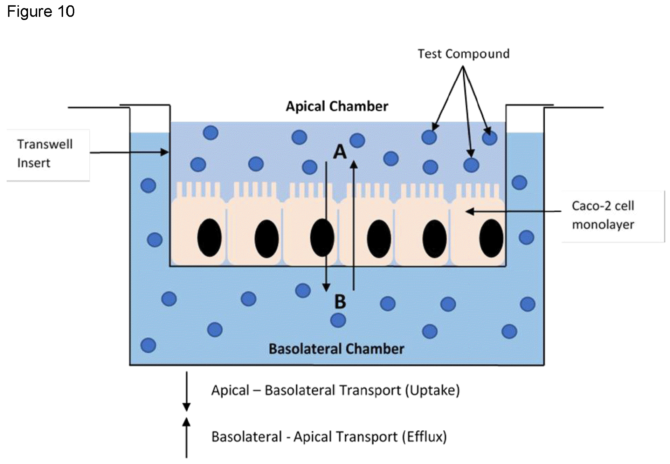

**Figure 10:** Diagram of how the Caco-2 flux assay is performed.

Sequences:

SEQ ID NO: 1 - generic synthetic peptide sequence
SEQ ID NO:2 - synthetic 13-mer peptide 1
SEQ ID NO:3 - synthetic 13-mer peptide 2
SEQ ID NO:4 - synthetic 13-mer peptide 3
SEQ ID NO:5 - sequence fraction N.aan_I15_R4
SEQ ID NO:6 - sequence fraction N.nka_I18_R4
SEQ ID NO:7 - sequence fraction N.naj_I25_R3
SEQ ID NO:8 - sequence fraction N.nub_I16_R4
SEQ ID NO:9 - sequence fraction N.nig_I19_R4
SEQ ID NO:10 - sequence fraction N.atr_I28_R3

## METHODS

### Collection of Cobra Venom:

Venoms were extracted from twelve cobra species using proprietary methods, collected into fresh vials, quantified, lyophilised and stored in a -20°C freezer until used.

### Purification of Cobra Venom Using 2 Dimensional HPLC:

Lyophilised cobra venoms were reconstituted in HPLC-grade $H_2O$ and the protein concentration determined using a DeNovix® DS11 spectrophotometer. Whole venoms were diluted to 100mg/ml in IE Buffer A and 100µl injected onto the Agilent® 1100 HPLC for separation via Ion Exchange chromatography. Venoms were separated using an increasing gradient of IE Buffer B over time. Peak detection parameters were set, and fractions collected. Fractions were lyophilised, reconstituted in RP Buffer A and reinjected onto the Agilent 1100 HPLC to be separated using reverse phase chromatography. Fractions were separated using an increasing gradient of RP Buffer B over time. From this process a panel of two-dimensional fractionated venom components were obtained and lyophilised. Fractions were resuspended, quantified via spectrophotometry and diluted to the same working protein concentration. Finally, fractions were plated out into low-protein binding 384 well plates, ready for use in assay screening.

### Rodent Cardiomyocyte and Cardiac Fibroblast Isolation:

Hearts were collected from euthanised 1-2-day old neonatal rat pups and transferred into filter sterilised ice-cold Hanks balanced salt solution (HBSS) containing 2% Pen-strep. Hearts were washed in the HBSS, any non-heart tissue removed and then cut into 1-3mm pieces and placed in 40ml filter sterilised, ice cold 0.1% trypsin/HBSS. The heart pieces in trypsin were place in a large ice bucket and incubated overnight at 4°C on a rocker with gentle agitation to allow the tissue to digest.

The following day, all trypsin was aspirated from the tissue and the tissue transferred evenly into 2ml Eppendorf® tubes. The tissue was then digested further using a 37°C 1mg/ml collagenase/HBSS (filter sterilised) solution. For each collagenase digestion, 2ml of enzyme buffer was added to all Eppendorf tubes and the tubes incubated in a 37°C heating block for 2mins with 400rpm agitation. The supernatant from the first two digestions was discarded. After each of the following digestion steps, the tissue was gently triturated using a pipette to release cells. Once trituration was complete the supernatant was retained, added to a tube containing 10ml cell culture media and stored on ice.

All cell containing supernatants were filtered through a 250µm cell strainer to remove any potentially undigested tissue and myocytes separated from cardiac fibroblast by spinning the supernatant at 1800g for 45mins through a Percoll® gradient. The band containing cardiomyocytes was collected separately from the band containing cardiac fibroblasts, both sets of cells were washed with HBSS and pelleted by centrifugation 3-times to remove Percoll buffer, resuspended in fresh culture media (DMEM:F12, 10% FCS, 2% PS) and the viable cell yield determine using a haemocytometer. Cells were plated in 384 well plates at a seeding density of $1x10^5$-$2x10^5$ cells/$cm^2$. All media was topped up to 50µl and cells returned to a humidified 37°C, 5% $CO_2$ incubator to settle and culture until spontaneously beating.

### Embryonic Chick Cardiomyocyte Isolation:

Hearts were collected from euthanised 12-13 day old embryonic chicks and transferred into filter sterilized ice-cold hanks balanced salt solution (HBSS) containing 2% Pen-strep. Hearts were washed in the HBSS to remove blood, any non-heart tissue removed and then cut into 1-3mm pieces and placed in 40ml filter sterilized, ice cold 0.05% trypsin/HBSS. The heart pieces in trypsin were place in a large ice bucket and incubated for 1h on a rocker with gentle agitation to allow the tissue to digest.

All trypsin was aspirated from the tissue and discarded, and the tissue transferred evenly into 2ml Eppendorf tubes. The tissue was then digested further using a 1mg/ml Collagenase II/0.1% Trypsin/HBSS (filter sterilized) solution. For each digestion, 2ml of enzyme buffer was added to all Eppendorf tubes and the tubes incubated in a 37°C heating block for 3mins with 400rpm agitation. The supernatant from the first two digestions was discarded. After each of the following digestion steps, the tissue was gently triturated using a Pasteur pipette to release cells. Once trituration was complete the supernatant was retained, added to a tube containing 10ml cell culture media (DMEM:F12, 10% FCS, 2% PS) and stored on ice.

All cell containing supernatants were filtered through a $250\mu m$ cell strainer to remove any potentially undigested tissue. The cells suspension was then pre-plated into a $75cm^3$ tissue culture flask at 37°C, 5% $CO_2$ for 2h to allow excess fibroblasts to settle out and stick. The remaining supernatant containing enriched myocytes were separated from remaining cardiac fibroblast by spinning the supernatant at 1800g for 1h through a Percoll gradient. The band containing immature cardiomyocytes was collected separately from the band containing residual cardiac fibroblasts, both sets of cells were washed with HBSS and pelleted by centrifugation 3-times to remove Percoll buffer, resuspended in fresh culture media (Fibroblast: DMEM:F12, 10% FCS, 2% PS; Chick myocytes: DMEM:F12, 10% Horse serum, 1% FCS, 2% PS) and the viable cell yield determine using a haemocytometer. Cells were plated in 384 well plates at a seeding density of $1x10^5$-$2x10^5$ cells/cm$^2$. All media was topped up to $100\mu l$ and cells returned to a humidified 37°C, 5% $CO_2$ incubator to settle and culture until spontaneously beating. Myocytes cells were seen to spontaneously beat 3 days after plating.

**Screening Purified Venom Fractions for Rat Cardiomyocyte and Cardiac Fibroblast Toxicity:**

Neonatal rat cardiomyocytes and cardiac fibroblasts were treated with purified venom fractions suspected of being cardiotoxins at doses of 12.5, 7.5, $6\mu g/ml$, with rat cardiomyocytes being treated at additional concentrations of 3.5, 2.5, 1 and $0.5\mu g/ml$ for 2hs in a humidified incubator at 37°C, 5% $CO_2$. After 2h resazurin was added to all dosed wells at a final working concentration of $160\mu M$. The change in fluorescence of the resazurin dye was monitored over a 5h timeframe using a BMG Fluostar plate reader. Percent inhibition of both cell types in response to each fraction at each dose were calculated and plotted graphically.

**Screening Purified Venom Fractions for Rat Erythrocyte Toxicity:**

Venom fractions were diluted to stock concentrations of $15\mu g/ml$ and $1\mu g/ml$ and plated out in triplicate ($100\mu l$/well) in v-bottom 96 well plates. A serial dilution of crude *Naja nigricollis* (N.nig) venom and DMSO were also plated out as toxicity controls.

Rat blood was collected post-mortem in PBS containing heparin. Erythrocytes were diluted in PBS and counted using a haemocytometer. Erythrocytes were diluted to a final concentration of $1x10^8$ cells/ml, spun at 300g for 3mins and the supernatant containing white blood cells removed. The erythrocyte pellet was resuspended in an equivalent volume of PBS. $100\mu l$ of resuspended cells were added to all fraction and toxicity control wells to give a final volume $200\mu l$/well ($1x10^7$ erythrocytes/well, $7.5\mu g/ml$ and $0.5\mu g/ml$ fraction working concentrations.

Erythrocytes were incubated in fractions at 37°C, 5% $CO_2$ to lyse or gravity settle for 5h. After 5h, supernatant was removed from all wells and plated in quadruplicate in 384 well plates ($30\mu l$/well). Absorbance of the plates were read at 620nm. All replicates were averaged and % erythrocyte lysis for both doses plotted graphically.

**Screening Purified Venom Fractions for Alterations to Cardiomyocyte Beat Rates:**

Neonatal rat cardiomyocytes were isolated as via standard protocol and seeded at a density of 100,000 cells/96 well. Once myocyte cultures were beating spontaneously in uniform sheets, they were videoed well-by-well using a microscope camera to determine pre-dose beat rates/min. Wells were dosed with suspected cardiotoxin fractions at doses of 12, 6, 3 and $1\mu g/ml$ in duplicate. Cells were incubated in each fraction in a humidified incubator at 37°C, 5% $CO_2$ for 1h. After 1h videos of the cells were re-recorded to obtain the post-dosing beat rate/minute. Fold changes in beat frequency were calculated for each dosed well pair.

**Assessing the Effects of Six Lead Fractions for Non-Target Species Effects Using Human IPSC-Derived Cardiomyocytes:**

Human IPSC-derived cardiomyocytes were purchased from Axol Biosciences and cultured as via their standard methodology. Briefly, cells were recovered from liquid nitrogen storage when required and the cells transferred dropwise into 10ml of pre-warmed (37°C) plating media (cardiomyocyte maintenance media + 10% FCS + 10$\mu$M ROCK inhibitor). Cells were centrifuged at 200x g for 5mins at room temperature. The supernatant was aspirated from the cell pellet and the cell pellet gently resuspended in 1ml plating media. Cells were counted using a haemocytometer and trypan blue staining. Cells were plated at a seeding density of 10-20,000 cells/well in fibronectin pre-coated 384 well plates. Cells were incubated at 37°C, 5% $CO_2$ in a humidified incubator overnight before the plating media was removed the following day and replace with fresh maintenance media (No FCS, no ROCK inhibitor). Partial media changes were performed every 2-days until cells formed a single continuous sheet of spontaneously beating cardiomyocytes (7-10 day in culture).

Once beating spontaneously, cells were treated with the six lead fractions diluted in maintenance media to final working concentrations of 7.5, 6, 3.5, 2.5 and 1$\mu$g/ml. 30$\mu$l of each fraction dilution was added to triplicate wells of cells and incubated for 2h at 37°C, 5% $CO_2$ in a humidified incubator. After 2h, resazurin dye at a final working concentration of 160$\mu$M were added to all wells and the change in fluorescence of the plate monitored over a 5h timeframe using a BMG Labtech Fluostar plate reader. Percent toxicity was calculated from triplicate wells at each dose and plotted graphically.

**Assessing the Thermal Stability of Six Lead Purified Venom Fractions:**

Thermal stability of the six lead fractions was investigated. Aliquots of each fraction were heated in a heat block to 37°C (unheated), 60°C, 80°C and 100°C before being used to treat rat neonatal cardiomyocyte cells at a dose of 6ug/ml. Cardiomyocyte toxicity was assessed after 24hrs of dosing at each temperature against a similar aliquot of each fraction that had been unheated before dosing. After 24h, 160$\mu$M Resazurin dye was added to all cells and the change in cardiomyocyte cell viability/metabolism monitored every hour up to 5h post dye addition. An additional measurement was recorded 24hs after the dye had been added.

**Mass Spectrometry Analysis of Six Selected Fractions:**

10$\mu$g of six fractions shown to display differing levels of cardiomyocyte toxicity and effects on beat rate underwent Intact Mass and Peptide Mapping mass spectrometry analysis (Peak Proteins Ltd, Alderley Park, Macclesfield). For intact mass spectrometry, 5$\mu$g of each lyophilised fraction sample was reconstituted in 40$\mu$l of 0.1% formic acid/5% acetonitrile. 10$\mu$l of the sample was loaded onto a Sciex® Exion™ liquid chromatography and a 5 min reverse phase gradient run, using a 0.1% formic acid Buffer A and a 0.1% formic acid/100% acetonitrile Buffer B. RP HPLC was performed with a flow of 500$\mu$l over a gradient, starting with 5% Buffer B increasing to 45% B over 3min, followed by a 95% B wash and an equilibration of 5% B. The column used was a Phenomenex Jupiter 5um, C4, 300A 50x2.1mm. Flow from the column was passed into the Sciex X500B mass spectrometer, set to collect data in positive ion mode. To enable ionisation of the eluate the source was set to 400°C, 5500V with gas at 50psi. A TOF mass window of 500-3000Da was collected, scanning at 0.5s. The X500B mass spectrometer was calibrated with a positive calibration mix and the error for the experiment was estimated to 0.5Da. The resultant total ion chromatogram (TIC) was deconvoluted using BioToolKit® software (Sciex).

For Peptide Mapping mass spectrometry, 5$\mu$g of each lyophilised sample was reconstituted in 50$\mu$l of 100 mM ammonium bicarbonate. 5$\mu$l of 100 mM DTT in 100 mM ammonium bicarbonate was added to each sample and the sample heated at 65°C for 30 min. 5$\mu$l of 500 mM iodoacetamide in 100 mM ammonium bicarbonate was added to all samples and the samples re-incubated in the dark at room temperature for 30 min. 10$\mu$l of 25ng/$\mu$l trypsin in 50 mM ammonium bicarbonate was added to each sample and they were incubated overnight at 37°C. 10$\mu$l of the complete digest was taken and mixed with 10$\mu$l of 0.1% trifluoroacetic acid (TFA). 10$\mu$l of mixed sample was loaded onto the Sciex Exion liquid chromatography and a 10 min reverse phase gradient run. A Phenomenex® Lunar™ 1.6 $\mu$m, PS C18, 100A 150x2.1mm column was used. Buffer compositions were as intact mass MS (0.1% formic acid Buffer A and a 0.1% formic acid/100% acetonitrile Buffer B). Flow was set to 300$\mu$l and a gradient performed, starting with 5% buffer B, increasing to 45% B over a 15 min duration, before a 95% buffer B wash and an equilibration at 5% B. Flow from the column was passed into the Sciex X500B mass spectrometer set to collect data in positive ion mode. To enable ionisation of the eluate the source was set to 400°C, 5500V with gas at 30psi. A TOF mass window of 300-1800Da was collected, scanning at 1.2s. Mass spec mass spec (MSMS) data was collected using an information Dependent Acquisition method, where up to 10 MSMS were collected per scan. The X500B was calibrated with positive calibration mix, the error for the experiment was estimated at 1ppm. The resultant data from the MSMS was analysed using Mascot (Matrix Science) using the Swiss-Prot database.

**SAR and Synthetic Peptide Design:**

Using the data obtained from the six fractions sent for mass spectrometry analysis, structure activity relationships (SAR) analyses were undertaken from which three short synthetic peptides were designed for further investigation. The three peptides were designed to exploit different characteristics of the six lead fractions that were investigated for their toxicity and beat-effect properties.

- **Peptide 1** - designed using the first 13 amino acids of fraction N.aan_I15_R4 which displayed no toxicity but caused increases to beat rate.

- **Peptide 2** - designed using the first 13 amino acids of N.aan_I15_R4 but substituted to contain amino acids important for both high beat rate and high toxicity at important loci identified by the SAR analysis.

- **Peptide 3** - designed using the first 13 amino acids of N.aan_I15_R4 but substituted to contain amino important for high beat rate and no toxicity at important loci identified by the SAR analysis.

**Screening Synthetic Peptides for Rat Cardiomyocyte, Chick Cardiomyocyte, Human IPSC-derived Cardiomyocytes and Cardiac Fibroblast Toxicity:**

Rat neonatal cardiomyocytes, Embryonic chick cardiomyocytes, human IPSC-derived cardiomyocytes and rat cardiac fibroblast were cultured. Synthetic Peptides 1, 2 & 3 were diluted to working concentrations of 100, 10, 1 and 0.1$\mu$g/ml in the cell culture media appropriate for the optimal growth of each cell type. Toxicity controls were also included in the experiment and were selected from whole venom and venom cardiotoxin fractions previously identified to display toxicity. Replicate wells of cells from each of the three cell types were also treated with 100$\mu$g/ml whole *N. nigricollis* venom and 10 & 1$\mu$g/ml of identified cardiotoxin fractions N.nka_I18_R4 and N.nig_I19_R4. All wells were incubated for 24h in each treatment. After 24h Resazurin dye was added to each well at a final concentration of 160$\mu$M and the change in fluorescence of the dye monitored and recorded over a 24h time period.

**Screening Synthetic Peptides for Alterations to Cardiomyocyte Beat Rates:**

The baseline beat rate of neonatal rat and human IPSC-derived cardiomyocytes to be treated with the three synthetic fractions were determined by video microscopy of each well in triplicate using a microscope camera. Average beats/min were calculated from the triplicate readings of three separate wells (n=9). Aliquots of each peptide were taken to treat neonatal rat cardiomyocyte cells at doses of 100, 10, 1 and 0.1$\mu$g/ml. Cells were returned to a humidified incubator at 37°C, 5% $CO_2$ for 2h. After 2h the beat rate of the treated cardiomyocyte cells was reassessed using the same method used to obtain the pre-dosing beat data. This process was repeated 24h after the addition of the synthetic peptides.
Embryonic chick cardiomyocytes were cultured until beating spontaneously. Cells were recorded by video microscopy and baseline beat rates were generated for cells pre-dosing with synthetic peptides. Synthetic peptides were diluted to final working concentrations of 100, 10, 1 and 0.1$\mu$g/ml in cardiomyocyte cell culture media. Chick cardiomyocytes were dosed for 2hs with the synthetics in a humidified cell culture incubator at 37°C, 5% $CO_2$, before beat rates were recorded again. Fold-changes in beat rate were calculated by dividing post-synthetic dosed beats/minute by the number of beats/min pre-dosing.

**Screening Synthetic Peptides for Thermal Stability:**

Thermal stability of the three designed synthetics peptides was investigated. The baseline beat rate of neonatal rat cardiomyocytes to be treated with the synthetic fractions were determined by video microscopy of each well in triplicate using a microscope camera. Average beats/min were calculated from the triplicate readings of three separate wells (n=9). Aliquots of each peptide were heated in a heat block at 100°C before being used to treat rodent cardiomyocyte cells at doses of 100, 10, 1 and 0.1ug/ml. Cells were returned to the incubator at 37°C, 5% $CO_2$ for 2h. After 2h the beat rate of the treated cardiomyocyte cells was reassessed using the same method used to obtain the pre-dosing beat data. This process was repeated 24h after the addition of the synthetic peptides. The data of the fold changes in cardiomyocyte beat rate following treatment with the heated synthetic fractions were compared to previously obtains fold-changes in beat rate of the unheated synthetic peptides to assess their thermal stability.

**Assessing Gut Permeability of Lead Short, Synthetic Peptide 1 Using a Caco-2 Permeability Assay (Bi-directional):**

Cells were seeded on to Millipore Multiscreen® Transwell® plates at 1 x 10$^5$ cells/cm$^2$. The cells were cultured in DMEM and media was changed every two or three days. On day 20, the permeability study was performed. Cell culture and assay incubations were carried out at 37 °C in an atmosphere of 5 % $CO_2$ with a relative humidity of 95 %. On the day of the assay, the monolayers were prepared by rinsing both basolateral and apical surfaces twice with Hanks Balanced Salt Solution (HBSS) at the desired pH warmed to 37 °C. Cells were then incubated with HBSS at the desired pH in both apical and basolateral compartments for 40 min to stabilise physiological parameters.

The dosing solutions were prepared by diluting test compound with assay buffer to give a final test compound concentration of 10μM or 100μM (final water concentration ≤1 % v/v). The fluorescent integrity marker lucifer yellow was also included in the dosing solution. Analytical standards were prepared from test compound water dilutions and transferred to buffer, maintaining a ≤1 % v/v water concentration. Assay buffer was composed of supplemented HBSS pH 7.4.

For assessment of apical to basolateral (A2B) permeability, HBSS was removed from the apical compartment and replaced with test compound dosing solution. The apical compartment insert was then placed into a companion plate containing fresh buffer (containing ≤1 % v/v Water). For assessment of B2A permeability, HBSS was removed from the companion plate and replaced with test compound dosing solution. Fresh buffer (containing ≤1 % v/v Water) was added to the apical compartment insert, which was then placed into the companion plate.

At 120 min, the apical compartment inserts and the companion plates were separated, and apical and basolateral samples diluted for analysis. Test compound permeability was assessed in duplicate. Compounds of known permeability characteristics were run as controls on each assay plate.

Test and control compounds were quantified by LC-MS/MS cassette analysis using a 7-point calibration with appropriate dilution of the samples. Cyprotex generic analytical conditions were used. The starting concentration (C0) was determined from the dosing solution and the experimental recovery calculated from C0 and both apical and basolateral compartment concentrations.

The integrity of the polarised cell monolayers throughout the experiment was checked by monitoring lucifer yellow permeation using fluorometric analysis. Permeation of this paracellular marker is low if monolayers have not been damaged. If a determined lucifer yellow Papp value was above acceptance limits (>1.0 cm/s x 10 $^6$) in one particular well, but the derived Papp result for test compound or positive control substrate in that well was qualitatively similar to that determined in the remaining replicate wells (within the lucifer yellow threshold) then, based upon the scientific judgement of the responsible scientist, the cell monolayer was considered acceptable. If this was not the case, then the result from the affected monolayer was excluded.

**Data Analysis:**

The determined concentration (nM ≡ pmol/mL) of each test sample receiver well is multiplied by the volume of the receiver compartment (apical = 0.09 mL, basolateral = 0.210 mL) to calculate the amount (pmol) of probe substrate permeating the monolayer. The amount determined in each receiver compartment is then divided by the incubation time (sec) to give the rate of transport of the test compound or positive control substrate, which is used to determine apparent permeability ($P_{app}$) according to the equation given below:

$$P_{app} = \left(\frac{dQ/dt}{C_0 \times A}\right)$$

Where dQ/dt is the rate of permeation of the drug across the cells, $C_0$ is the donor compartment concentration at time zero and A is the area of the cell monolayer. $C_0$ was obtained from analysis of the dosing solution.

An efflux ratio (ER) was calculated from mean A to B and B to A data. This is derived from:

$$ER = \frac{P_{app(B-A)}}{P_{app(A-B)}}$$

Percentage recovery (mass balance) of the test compound or positive control substrate was calculated by dividing the sum of the amount (pmol) in the receiver and donor compartments post-incubation by the amount in the initial donor solution at time zero ($C_0$) and expressing as a percentage.

Four control compounds were screened alongside the test compounds, atenolol (human absorption 50 %), antipyrine (human absorption 97 %), talinolol (a P-gp substrate) and estrone 3-sulfate (a BCRP substrate).

**RESULTS**

**Cardiomyocyte and Cardiac Fibroblast Toxicity Assays:**

At a dose of 12.5μg/ml, fourteen and eleven of the purified synthetic fractions displayed greater than 50% toxicity against rat cardiomyocytes and cardiac fibroblasts respectively (Figure 1A). Reducing the dose of the purified venom fractions resulted in the loss of fibroblast toxicity in all but one of the tested purified fractions (N.naj_I24_R2). Eight of the purified fractions were still found to cause greater than 50% toxicity to rat cardiomyocytes at a dose of 6ug/ml (Figure 1C). The data shows that by selecting the correct dose, selectivity for cardiomyocytes over cardiac fibroblasts can be achieved. Interestingly, purified fractions from the venom of *Naja annulifera* caused no toxicity to either cardiomyocytes or cardiac fibroblasts at any of the three tested doses.

Profiling of a panel of purified fractions, suspected to be cobra cardiotoxins from their HPLC peak elution times and profiles, identified that the fractions displayed a wide range of differing levels of cytotoxic effects against both neonatal rat cardiomyocyte and neonatal rat cardiac fibroblast cells. The data also identified that the window between a toxic and nontoxic dose of many of the fractions was relatively narrow, with toxicities of many fractions approaching 90-100% at a dose of 12.5µg/ml and with toxicity completely abolished around 1-2.5µg/ml. Overall, neonatal rat cardiomyocyte cells displayed greater susceptibility to many of the screened fractions than their dosed cardiac fibroblast counterparts. Fractions isolated from the venom of the snouted cobra *Naja annulifera* (N.aan) displayed very little or no cytotoxic effects against either cardiomyocyte or cardiac fibroblast cells. Interestingly, this was the only fractionated cobra venom for which all the screened fractions displayed no toxic effects at any of the tested doses.

Referring to Figure 1C, a dose of 6µg/ml displayed the greatest selectivity in toxic effects between the target rat cardiomyocytes and the non-target cardiac fibroblast control cell groups, with cardiac fibroblast toxicity all but abolished following treatment with all fractions apart from one (N.naj_I24_R2). Whilst fibroblast toxicity was completely abolished at 6µg/ml, rodent cardiomyocyte cytotoxicity of 40% or greater were still observed with 12 of the tested fractions, with 8 of these displaying greater than 70% toxicity.

Additional selectivity's were still observed with other fractions at higher tested doses, with good selectivity between cardiomyocytes and cardiac fibroblast achieved with fraction N.nub_I16_R3 at a dose of 7.5µg/ml (Figure 1B) and with fraction N.atr_I23_R3 at the highest tested dose of 12.5µg/ml (Figure 1A).

This initial toxicity screening data supports the hypothesis that components of cobra venoms could be isolated and used for the selective stressing of rodent cardiomyocyte cells and therefore have the potential for application as a novel, unique rodenticide exploiting a previously uninvestigated mechanism of action. The selectivity data showing that the fractions are more selectively toxic to cardiomyocytes over cardiac fibroblast, which are also found in heart tissue, is encouraging and suggests the potential for the development of something that is highly specific and targeted to a single cell type.

It was decided that the additional screening of these purified fractions against another completely morphologically distinct cell type, rat erythrocytes, would be important in assessing whether fractions may display unwanted compounding effects such as haemolytic properties which could be detrimental to the effectiveness of a synthetically designed version of these fractions.

**Erythrocyte Toxicity Assay:**

Referring to Figure 2, the erythrocyte lysis assay showed that none of the purified venom fractions caused the death of rat erythrocytes at either a dose of 0.5 or 7.5µg/ml. Interestingly, doses of 7.5µg/ml of some of the purified fractions had been shown previously to display toxicity to either rat cardiomyocytes, rat cardiac fibroblasts or both, and so adds further evidence to a selectively targeted mechanism of action. Crude *Naja nigricollis* venom was used as a control for erythrocyte toxicity and was shown to be capable of lysis rat erythrocytes at doses of 50µg/ml and 5mg/ml.

No erythrocyte toxicity was observed with any of the tested fractions at a top dose of 7.5µg/ml, a dose previously found to be cardiomyocyte toxic for a large number of the tested fractions. This lack of toxicity supports that all tested fractions do not display haemolytic properties, therefore suggesting that the cardiomyocyte cell toxicity previous observed in response to treatment with these purified fractions is more likely to be mechanistic in action rather than a generalised necrosis or apoptosis triggering effects. Treatment with crude venom from Cobra *Naja nigricollis* as a control determined that it is possible for venoms from cobras to cause erythrocyte lysis, supporting the findings that these purified fractions do not.

**Screening Purified Venom Fractions for Alterations to Cardiomyocyte Beat Rates:**

After 1h of dosing all screened fractions (with the exception of those from *Naja annulifera* (N.aan) were toxic to the rat cardiomyocytes at 12µg/ml, and so no post-dosing beat data could be obtained. Dosing with other concentrations of each fraction gave varying fold-increases to beat rate (see Table 1 below), confirming that cobra venom fractions can indeed cause increases to the beat rate of rodent cardiomyocyte cells. Of the screened fractions, eighteen caused increases to the beat rate of rat cardiomyocytes in at least one tested concentration. Of the three fractions that did not cause increases to beat rate (N.atr_I5_R5, N. kao_I18_R3 and N.nig_I4_R3), there was also no observable cytotoxicity seen with the previous Resazurin assays (See Figure 1) at any of the tested concentrations, and so taken together it is unlikely that these fractions actually do contain any compounds that actively effect cardiomyocyte functions or viability.

Most fractions were found to cause the greatest increases to rat cardiomyocyte beat rate at doses of either 6 or 3.5μg/ml, with drop offs in beat rate at higher doses, which can likely be attributed to cellular cytotoxicity and death. Fractions displayed greatly different increases in rat cardiomyocyte beat rate with a lowest maximum fold-increase of 2-fold observed with N.nub_I17_R2 and a highest fold increase of 13.75-fold observed with N.naj_I25_R3, showing that the actives in each fraction have vastly differing levels of activity against the cells.

Table 1

| Fraction | Dose (μg/ml) | Highest Achieved Fold Increases in Beat Rate |
|---|---|---|
| N.aan_I15_R4 | 6 | 3.50 |
| N.aan_I16_R6 | 12.5 | 2.20 |
| N.aan_I19_R8 | 3.5 | 2.75 |
| N.aan_I22_R6 | 12.5 | 12.60 |
| N.atr_I5_R5 | - | - |
| N.atr_I23_R3 | 3.5 | 5.00 |
| N.atr_I28_R3 | 6 | 5.35 |
| N.nub_I16_R3 | 3.5 | 11.40 |
| N.nub_I17_R2 | 6 | 2.00 |
| N.nub_I19_R3 | 6 | 7.86 |
| N.nka_I18_R4 | 6 | 7.00 |
| N.kao_I18_R3 | - | - |
| N.kao_I20_R3 | 3.5 | 5.00 |
| N.sia_I20_R3 | 6 | 3.60 |
| N.sia_I21_R2 | 6 | 2.46 |
| N.nig_I4_R3 | - | - |
| N.nig_I19_R4 | 1 | 6.25 |
| N.nig_I24_R4 | 3.5 | 2.20 |
| N.naj_I23_R5 | 6 | 4.30 |
| N.naj_I24_R2 | 6 | 11.13 |
| N.naj_I25_R3 | 3.5 | 13.75 |
| - Fractions did not cause increases to beat rate at any of the tested concentrations. | | |

**Selection of Six Lead Fractions and confirmation of their Toxicity and Cardiomyocyte Beat Rate Effects:**

Using the cardiomyocyte and cardiac fibroblast toxicity data, and the data obtained for changes in beat rate pre- and post-dosing, six fractions were selected to take forward for further characterisation. Fractions with varying myocyte and non-myocyte toxicity profiles were taken forward to allow for the identification via mass spectrometry and SAR of amino acid residues important for cardiomyocyte toxic effects specifically. The yield of each purified fraction was also considered as an attributing factor for selection.

The six fractions were selected based on the following justifications:

**N.aan_I15_R4:** Showed no visibly or detectible toxicity to either rat cardiomyocytes or cardiac fibroblast at doses up to 12.5μg/ml. Caused greater than 3.5-fold increases to beat rate at a dose of 6μg/ml. Has no cytotoxicity but can increase the contraction rate of rat cardiomyocytes.

**N.atr_I28_R3:** Showed rat cardiomyocyte and cardiac fibroblast toxicities at doses of 3μg/ml and above and 7.5μg/ml and above respectively. Caused greater than 5-fold increases to beat rate at a dose of 6μg/ml. High cytotoxicity to specific and non-specific cell types and causes strong increases to the contraction rate of rat cardiomyocytes.

**N.nub_I16_R3:** Showed rat cardiomyocyte and cardiac fibroblast toxicities at doses of 7.5$\mu$g/ml and above and 12.5$\mu$g/ml respectively. Caused greater than 6-fold increases to beat rate at a dose of 6$\mu$g/ml. Lower cytotoxicity to specific and non-specific cell types than seen with N.atr_I28_R3 (higher doses required) and causes strong increases to the contraction rate of rat cardiomyocytes.

**N.nka_I18_R4:** Showed rat cardiomyocyte and cardiac fibroblast toxicities at doses of 3$\mu$g/ml and above and 7.5$\mu$g/ml and above respectively. Caused greater than 7-fold increases to beat rate at a dose of 6$\mu$g/ml. High cytotoxicity to specific and non-specific cell types and caused extremely high increases to the contraction rate of rat cardiomyocytes.

**N.nig_I19_R4:** Showed rat cardiomyocyte and cardiac fibroblast toxicities at doses of 7.5$\mu$g/ml and above and 12.5$\mu$g/ml respectively. Caused greater than 5.5-fold increases to beat rate at a dose of 6$\mu$g/ml. Similar cytotoxicity profile to specific and non-specific cell types as that seen with N.nub_I16_R3 and caused similar increases to the contraction rate of rat cardiomyocytes.

**N.naj_I25_R3:** Showed rat cardiomyocyte and cardiac fibroblast toxicities at doses of 6$\mu$g/ml and above and 12.5$\mu$g/ml respectively. Caused greater than 8.5-fold increases to beat rate at a dose of 6$\mu$g/ml. More toxic to rat cardiomyocytes than N.nub_I16_R3 and N.nig_I19_R4 but with similar cardiac fibroblast toxicities. Caused extremely high increases to the contraction rate of rat cardiomyocytes.

**Assessing the Effects of Six Lead Fractions for Non-specific Species Effects Using Human IPSC-derived Cardiomyocytes:**

Human induced pluripotent stem cell (IPSC) derived cardiomyocytes were purchased from Axol Bioscience and cultured as directed. After 48hrs cells began to display the formation of a spontaneous beating monolayer of cells. After 7days in culture, cells were ready to be used to assess potential human cardiomyocyte toxicity in response to treatment with our six selected cardiotoxin fractions.

Human IPSC derived cardiomyocytes were dosed in triplicate with a serial dilution of each of the six venom fractions (7.5, 6, 3.5, 2.5 and 1ug/ml) for 2hs at 37°C, 5% $CO_2$ before resazurin dye was added and the change in colour and fluorescence monitored for the following 5hrs. Percentage toxicity was calculated for each fraction at all doses and plotted graphically (Figure 3).

Five of the six selected fractions displayed cytotoxic activity against human IPSC-derived cardiomyocytes, with only fraction N.ann_I15_R4 continuing to display low levels of cytotoxicity across all five of the tested doses. Obvious dose dependent effects were observed in response to treatment with the five other purified venom fractions, with higher doses eliciting higher levels of cardiomyocyte toxicity.

Resazurin data showed there is a nice dose response profile in human IPSC cardiomyocytes in response to treatment with five of the selected fractions (see Figure 3). As seen previously in neonatal rodent cardiomyocytes, there appears to be no observable cardiomyocyte cellular toxicity in response to treatment with fraction N.aan_I15_R4.

The toxicity data for the human IPSC cells appears to suggest a higher level of toxicity in these cells in response to some fractions than previously observed in rat neonatal cardiomyocytes, with cells displaying partial toxicities at doses lower than seen with the rodent myocytes. However, fractions N.atr_I28_R3 and N.nka_I18_R4 showed a reduction in toxicity at 3.5$\mu$g/ml in human IPSC-derived cells compared to the toxicity levels previously seen in rodent cardiomyocytes.

It is worth noting that these cells are not mature cardiomyocytes, but rather human stem cells that have been programmed to differentiate into cardiomyocyte-like cells. The human IPSC cells have a different cellular origin to the rodent cardiomyocyte cells isolated directly from the animal. As they require a fine balance of growth factors to trigger their differentiation, they may display a greater susceptibility to environmental stressors and changes.

**Assessing the Thermal Stability of Six Selected Purified Venom Fractions:**

Referring to Figure 4, overall, all purified fractions displayed good thermal stability profiles, with strong cytotoxic activity retained following treatment of the fractions at temperatures as high as 100°C. Slight reductions to toxicity were observed (around 5-10%) following the treatment of fractions N.nub_I16_R3 and N.atr_I28_R3 at 100°C when compared to their activity at 37 °C (without heat treatment), however these reductions were considered to be negligible. Fraction N.ann_I15_R4 continued to display lower than average levels of cytotoxicity at all the four tested temperatures consistent with the finds of the previous toxicity screens.

Analysis of thermal stability of the selected six fractions revealed that they appear to be thermally stable to temperatures as high as 100°C with little or no drop off in their cardiomyocyte toxicity (see Figure 4). Fractions with slightly lower outright toxicity to start with (N.ann_15_R4 and N.nub_I16_R3) showed a slightly more pronounced drop off in activity, however this still only amounted to around a 10% loss in cell cytotoxicity at the highest heated temperature of 100°C. Fraction N.ann_I15_R4 continued to show low levels of cardiomyocyte toxicity at all tested temperatures. However, this is not surprising as it has continually been shown to be far less outright cytotoxic to rat cardiomyocyte cells than any of the other five selected fractions. There were no significant changes in the activity of fractions N.nka_I18_R4, N.nig_I19_R4 and N.naj_I25_R3 in response to treatment with any of the tested temperature, with the same cytotoxic activity observed at 100°C as with 37°C. The data suggests that the active components in each fraction are capable of withstanding temperatures in excess of 100°C with no or little change in their stability and activity.

The active compound in the final product needs to be able to display good thermal stability if it is to withstand incorporation into hot liquid wax (~70°C) and retain its active properties after the cooling and setting process.

**Mass Spectrometry Identification of Six Selected Fractions:**

Six fractions, shown to display cardiotoxic effects in rat neonatal cardiomyocytes were selected and sent for mass spectrometry analysis. Intact mass analysis combined with peptide mapping confirmed that all six selected fractions were cardiotoxins, matching obtained sequence to cardiotoxins reported in Uniport and NCBI protein databases. Three of the selected fractions provided high percentage match coverage to cardiotoxins already present in the database (suggesting they are most likely these specific cytotoxins).

Certain regions of sequence are highly conserved across all cardiotoxins, most likely for structural integrity.

Mass Spectrometry-matched amino acids in **bold.** Conserved amino acids across cardiotoxin sequences in Uniprot (but not matched in obtained mass spectrometry data) in underlined. Grey highlighting denotes amino acid substitutions.

N.aan_I15_R4: (matched to *Naja annulifera* cytotoxin 1) ≈91.6% match:

**LKC**HK**LVPPV WKTCPEGKNL CYKMFMVSTS TVPVKRGCID VCPKDSALVK YVCCSTDKCN** [SEQ ID NO:5]

N.nka_I18_R4: (matched to *Naja naja* cytotoxin 8) ≈96.6% match:

**LKCNKLIPLA YKTCPAGKDL CYKMYMVSDK TVPVKRGCID VCPKNSLLVK YECCNTDRCN** [SEQ ID NO:6]

N.naj_I25_R3: (matched to *Naja naja* cytotoxin 10) ≈75% match:

**LKC**NK**LVPLF YKTCPAGKDL CYKMYMVATP** K**V**P**VKRGCID VCPK**S**SLL**VK **YVCCNTDRCN** [SEQ ID NO:7]

Whilst the other three sequences yielded lower percentage matches due to lack of information of characterised cardiotoxins from those species in the database, it was still possible to predict the most likely sequences. By using the sequence data obtained from mass spectrometry, the sequence of cardiotoxins (in uniport/NCBI) from closely related species, and the sequences of conserved regions across all cardiotoxin sequence structures, it was possible to account for the likely sequence of some of the amino acids in these cardiotoxins. Only amino acids shown in the database to have high variability at a particular loci and no mass spec sequence data, remain unconfirmed (black amino acids).

Matched amino acids in **bold.** Conserved amino acids but not matched to peptides in underlined. Grey highlighting denotes amino acid substitutions.

N.nub_I16_R4: (matched to *Naja pallida* cytotoxin 1) ≈65% match:

**LKCNQLIPPF WK**TCP**KGKNL CYK**MTMR**AAP MVPVKRGCID VCPK**S**SLL**IK **YMCCNTDKCN** [SEQ ID NO:8]

N.nig_I19_R4: (match to *Naja mossambica* cytotoxin 4 with amino acid substitutions) ≈43.3% match:

**LKC**NKLI**P**IA Y**KTCP**EGKNL **CYK**MMLASKK MV**P**V**KRGCID VCPKDSALVK** YV**CC**ST**DR**C**N** [SEQ ID NO:9]

N.atr_I28_R3: (match to *Naja atra* cytotoxin 5 with amino acid substitutions) ≈46.6% match:

**LKC**NKL**VPLF** Y**KTCP**A**GKNL CYK**MFMVSNL **TVPVKRGCID VCPK**S**S**L**L**VK YV**CC**NT**DR**C**N** [SEQ ID NO:10]

**SAR and Synthetic Peptide Design:**

Using the above sequences, Structure Activity Relationship (SAR) analyses were undertaken to determine which amino acid residues were likely the most important for causing the changes observed in cardiomyocyte toxicity, non-specific cardiac fibroblast toxicity and changes in the fold-increase in beat frequency in cardiomyocytes post-dosing.

SAR identified the highlighted amino acids shown in Figure 5 as important for cardiomyocyte toxicity. SAR revealed that most of the important amino acids are situated within the first 30 amino acids of the sequence, supporting that loop 1 and the start of loop 2 of the cardiotoxin structure are the most important for cardiomyocyte toxicity. Using this information, it was proposed as the region of the cardiotoxins sequence from which short active synthetic peptides would be designed.

Three short synthetic peptides were designed using the SAR analysis. Peptide 1 was designed using the first 13 amino acids of cardiotoxin N.aan_I15_R4 (matched by mass spec analysis to *Naja annulifera* cardiotoxin 1) as this was the only investigated cardiotoxin that displayed large increases to cardiomyocyte beat rate without causing cytotoxicity and so made a good choice as a starting scaffold to ensure synthetic peptides with low levels of outright toxicity:

Synthetic 1: LKCHKLVPPVWKT [SEQ ID NO:2]

Synthetic Peptide 2 was designed using the first 13 amino acids of cardiotoxin N.aan_I15_R4 as the overall peptide framework, but with those amino acids shown by SAR analysis to cause high beat rate and high toxicity substituted into the sequence.

Synthetic 2: LKC**N**KLIP**LAY**KT [SEQ ID NO:3]

Amino acids in bold are those that SARs showed as being important for HIGH toxicity. Underlined amino acids are those that SARs showed as being important for HIGH beat rate.

Finally, Synthetic 3 was designed using the first 13 amino acids of cardiotoxin N.aan_I15_R4 as the overall peptide framework, but with those amino acids shown by SAR analysis to only be important for high beat rate (no toxicity) in the other investigated cardiotoxins substituted into the sequence. The substitution of these amino acids into the sequence was found to give the peptide poor solubility in water and so an additional lysine (K) was added to the end of the peptide sequence to improve solubility.

Synthetic 3: LKC**H**KLIPIA**W**KTK [SEQ ID NO:4]

Amino acids in bold are those that SAR demonstrated as being important for LOW toxicity. Underlined amino acids are those that SAR displayed as being important for LOW beat rate.

**Screening Synthetic Peptides for Rat Cardiomyocyte and Rat Cardiac Fibroblast Toxicity:**

All three short, synthetically designed peptides displayed no cytotoxic effects against either cardiomyocytes or cardiac fibroblasts at any of the four screened doses (0.1, 1, 10 and 100μg/ml). Treatment with crude *N. nigricollis* venom at a dose of 100μg/ml and purified fractions N.nka_I18_R4 and N.nig_I19_R4 at a dose of 10μg/ml continued to display high levels of toxicity (≈ 90%) in both rat cardiomyocytes and cardiac fibroblasts.

After 24hs of treatment with the three synthetic peptides, there was no observable toxicity to either neonatal rat cardiomyocytes (see Figure 6A) or cardiac fibroblasts (see Figure 6B). This toxicity data demonstrates that the three synthetic peptides displayed no cytotoxic activity against both cardiomyocyte and non-myocyte cell types.

The synthetic design process, producing peptides that represent only the first finger of the native cardiotoxins in conjunctions with using the amino acid sequence from fraction N.ann_I15_R4 (non-cytotoxic cardiotoxin) as the scaffold for building all three synthetics, has helped in the elimination of the toxicity previously seen with the other cytotoxic fractions. 100μg/ml whole *N. nigricollis* venom and 10μg/ml of cardiotoxin containing fractions N.nka_I18_R4 and N.nig_I19_R4 continued to display high levels of cytotoxicity against both tested cell types.

This data has shown that by designing shortened synthetic versions of the peptides, containing amino acids from loop 1 of the cardiotoxin structure only, undesirable cytotoxic effects that are observed with the full length cardiotoxins were engineered out.

**Selectivity Screening: Screening Synthetic Peptides for Human IPSC Cardiomyocyte Toxicity:**

Referring to Figure 7, all three short, synthetically designed peptides displayed no cytotoxic effects against either type of cardiomyocytes at any of the 4 screened doses (0.1, 1, 10 and 100$\mu$g/ml). Treatment with crude *N. nigricollis* venom at a dose of 100$\mu$g/ml and purified fractions N.nig_I19_R4 & N.nka_I18_R4 at doses of 10$\mu$g/ml continued to result in high levels of toxicity ($\approx$ 90%) against both species myocytes.

The three synthetic fractions were screened against commercially available human IPSC-derived cardiomyocyte cells and isolated embryonic chick cardiomyocyte cells to determine whether they displayed any human or avian specific cytotoxicity. After a 24h dosing timeframe, no outright cytotoxicity was observed in either the human IPSC (Figure 7A) or embryonic chick cardiomyocytes (Figure 7B) in response to any of the three synthetically designed peptides at any of the tested doses.

Toxicity was still observed in these cells in response to treatment with the two native cardiotoxin fractions, previously purified and crude venom from the cobra *Naja nigricollis.*

The data indicates that human cardiomyocyte cell cytotoxicity is not a characteristic of the synthetic peptides designed at this stage.

**Screening Synthetic Peptides for Alterations to Rodent and Human IPSC Cardiomyocyte Beat Rates:**

Referring to Figure 8, all three peptides caused greater increases in fold-beat rate in rat cardiomyocytes than the human IPSC-derived cardiomyocytes, where little to no increases were seen in response to treatment with any of the doses. Synthetic peptide 1 caused the greatest fold increases to beat rate in rat cardiomyocytes, with a dose of 100$\mu$g/ml eliciting an 8-fold increase to measured beat rate. Synthetic peptides 2 and 3 caused peak increases of 3.5- and 3-fold respectively in rat neonatal cardiomyocyte beat rates.

Neonatal rodent cardiomyocytes and human IPSC-derived cardiomyocytes were dosed with the three synthetically designed peptides and changes to their beat rates in response to dosing for 2hs was assessed. Following 2hs of dosing at 100, 10 1 and 0.1$\mu$g/ml rodent cardiomyocytes saw increases to beat rate in response to all three synthetic fractions at all tested doses. Synthetic 2 (Figure 8B) showed increases of around three-fold that of the pre-dosing beat rate in response to all four tested concentrations, whilst synthetic 3 (Figure 8C) saw increases to beat rate of around two-fold for the three lower tested concentrations, with an increase to three-fold following treatment with the highest tested dose of 100ug/ml. Synthetic 1 (Figure 8A) showed the greatest fold-increases to beat rate of the three tested synthetic compounds, with fold-increases to beat rate of 3.5-fold, 4-fold, 5-fold and 8-fold at 0.1, 1, 10 and 100$\mu$g/ml respectively. Increases to heart rate of three-fold or greater in response to the synthetic fractions are likely to have catastrophic repercussions for the efficient functioning of the rodent heart.

The effects of the three synthetic peptides were also assessed on human IPSC-derived cardiomyocyte cells, to determine whether they displayed any activity against an off-target species. After dosing for the same 2h timeframe and at the same four doses investigated in the rat cells (100, 10, 1, 0.1$\mu$g/ml) the synthetic peptides appeared to display little or no effects against human IPSC-derived cardiomyocytes compared to the effects that were observed against rat cardiomyocytes. All three synthetic peptides caused no increases to the beat rates of human IPSC cardiomyocytes at doses of 0.1, 1 and 10$\mu$g/ml. Synthetic peptides 1 and 2 appeared to cause slight increases to the beat rate of the human cardiomyocytes of 1.5-fold the pre-dosing beat rate. However, with the much larger increases observed in the rodent cardiomyocyte cells at the same dose using synthetic peptide 1, there is the potential for selecting a dose that displays selectivity towards rodent cardiomyocytes only, by exploiting the large window of difference observed between the two species.

All synthetics were not found to cause increases to the beat rate of chick cardiomyocytes at any of the tested concentrations (Figure 8A-C), as also previously seen with human IPSC-cardiomyocytes. The data generated into the effects of the three synthetically designed peptides show that the peptides display preferential activity against rodent cardiomyocytes when compared to their human or chick-derived counterparts. These differences are promising in terms of reducing the potential issue of side-effects occurring in large mammalian or avian off-target species, should accidental exposure to these novel peptides occur, and suggests scope for an even larger dosing window between target and non-target species when circulating blood volume differences and heart size are taken into consideration.

**Screening Synthetic Peptides for Thermal Stability:**

Referring to Figure 9, all three synthetic peptides showed a slight loss in activity in response to heating to 100°C when compared to the same beat rates obtained following treatment with unheated fractions, however the fractions still retained the majority of their activity despite being heated to 100°C. Synthetic peptide 1 (Figure 9A) still performed the best in terms of increases to beat rate, with the thermally treated fraction still causing 7-fold, 4-fold, 3-fold and 3-fold increases to beat rate following treatment with 100, 10, 1 and 0.1$\mu$g/ml of the peptide respectively.

Synthetic 2 (Figure 9B) continued to cause approximately 3.5-fold increases in beat rate at 100μg/ml and seemed to show greater thermal stability at higher concentrations. Reductions to activity were observed in peptide 2 with doses of 10, 1 and 0.1μg/ml, however all doses still caused 2.5-fold or greater increases to rat neonatal cardiomyocyte beat rates. Synthetic 3 (Figure 9C) displayed the lowest fold-increases to beat rate following heating, with maximum fold-increase reach at 2.5-fold.

Assessing the beat increase and thermal stability data of all three peptides, synthetic 1 appeared to perform best, causing the greatest increases to rat cardiomyocyte beat rates, without causing similar increases to beat rate in human IPSC-derived cardiomyocytes. Peptide 1 also continues to display good activity against rat neonatal cardiomyocytes when heated to a temperature of 100°C, showing that its displays fantastic thermal stability characteristics. Taking all the data of the three synthetic peptides into consideration, synthetic peptide 1 was selected for further assessment of its gut permeability and sent for Caco-2 assay analysis.

**Assessing gut permeability of lead short, synthetic peptide 1 using a Caco-2 permeability assay:**

As illustrated in Figure 10, Caco-2 cells were grown to a monolayer in a Transwell insert on a permeable membrane. The insert was inserted into the well of a plate to form an Apical chamber (above cells) and basolateral chamber (below cells), with a permeable membrane between the two layers. Compound was then added to either the apical or basolateral chamber and the passage of it monitored into the other chamber. Transfer of the test compound from the apical to basolateral chamber was indicative of cellular uptake, simulating transition of the compound from the intestinal lumen to the bloodstream by uptake receptors (Direction A). Transfer of the compound from the basolateral to apical chamber was indicative of compound efflux and suggests that, whilst the compound may cross the intestinal wall, it was transported back into the intestinal lumen by efflux receptors found on the surface of the intestinal cells (Direction B). An Efflux ratio was then calculated from the values obtained for A2B and B2A transmission. A good compound for gut absorption should display permeability in the A2B direction but low transmission in the B2A direction.

The control compounds behaved as expected in the assay and all Papp and efflux ratio values were within acceptable limits where available (Table 2).

Table 2: Caco-2 permeability positive control data

| Compound | | $P_{app}$ (x $10^{-6}$ cms$^{-1}$) | | | SD | n | Mean % Recovery | ER |
|---|---|---|---|---|---|---|---|---|
| | | Rep 1 | Rep 2 | Mean | | | | |
| Antipyrine | A2B | 47.2 | 46.9 | 47.1 | 0.255 | 2 | 90.5 | 0.840 |
| | B2A | 38.0 | 41.1 | 39.6 | 2.15 | 2 | 87.8 | |
| Atenolol | A2B | 0.220 | 0.250 | 0.235 | 0.0213 | 2 | 88.5 | 1.89 |
| | B2A | 0.395 | 0.497 | 0.446 | 0.0720 | 2 | 95.7 | |
| Talinolol | A2B | 0.201 | 0.236 | 0.218 | 0.0245 | 2 | 57.7 | 45.3[a] |
| | B2A | 7.91 | 11.8 | 9.88 | 2.78 | 2 | 78.8 | |
| Estrone 3-sulfate | A2B | 0.225 | - | 0.225 | - | 1 | 68.6 | 155 [b] |
| | B2A | 33.1 | 36.6 | 34.9 | 2.47 | 2 | 85.7 | |
| Rep = Replicate; n = number of replicates; ND = Not detected; NA = Not applicable<br>a Test compound sample concentration from receiver compartment of replicate 1 below LQS A2B.<br>b Test compound not detectable in receiver compartment of replicate 2 A2B. | | | | | | | | |

Data for DMVTL061120 (synthetic peptide 1) at 10μM showed the compound could not be detected at lower concentrations in the standard curve and therefore the samples from the Caco-2 experiment concentration was increased to 100μM. Data for DMVTL061120 at 100μM is shown in Table 3.

**Table 3:** Caco-2 permeability DMVTL061120 data (synthetic peptide 1)

| | $P_{app}$ (x $10^{-6}$ cms$^{-1}$) | | | SD | n | Mean % Recovery | ER |
|---|---|---|---|---|---|---|---|
| | Rep 1 | Rep 2 | Mean | | | | |
| A2B | 6.48 | 2.73 | 4.60 | 2.65 | 2 | 45.5 | 0.137 |
| B2A | 0.681 | 0.584 | 0.633 | 0.0688 | 2 | 96.6 | |
| Rep = Replicate; n = number of replicates | | | | | | | |

DMVTL061120 (synthetic peptide 1) gave mean Papp values of 4.60 and 0.633 x$10^{-6}$cms$^{-1}$ for the A2B and B2A directions respectively. The efflux ratio was 0.137 and the mean percent recovery A2B and B2A was 45.5 and 96.6 % respectively. The efflux ratio of 0.137 suggests the compound is not a substrate for efflux transporters but could be a possible substrate for uptake transporters because the efflux ratio was <1.

The findings of the Caco-2 flux assay suggest that synthetic peptide 1 may be readily taken up and absorbed by cells of the intestinal tract. The data in the apical to basolateral (A2B) direction suggest that the peptide could be a possible substrate for uptake transports and, as such, should be capable of efficiently traversing the intestinal lining for uptake into the blood stream. There was some loss in recovery of the peptide in the A2B direction, suggesting that some of the peptide may be interacting with or being retained or internalised by the intestinal cells themselves.

The data in the basolateral to apical (B2A) direction showed little detection of the peptide passing in this direction, and so supports that the peptide was not readily effluxed from the basolateral cellular environment by efflux transporters. This is important information as it suggests that, once internalised and across the lining of the intestinal wall, the peptide is not readily pumped back into the intestinal lumen, and so has a greater chance of being absorbed into the bloodstream once absorbed through the intestine lining.

The addition of lucifer yellow permeation, using fluorometric analysis, during the experimentation allowed for the monitoring of the integrity of the polarized cell monolayer. Lucifer yellow monitoring allowed for the determination that toxicity to the Caco-2 cells by synthetic peptide 1 was not observed at a dose of $100\mu M$. The fact that the cell monolayer remained intact upon treatment with the synthetic peptide is key, as it suggests the synthetic peptide is unlikely to cause gastrointestinal cell damage, leading to stomach upset when ingested. And so, it can be inferred that the ingestion of peptide 1 is unlikely to result in bait shyness from its rodent targets.

In summary, SAR identified amino acids important for cardiomyocyte toxicity and revealed that most of the important amino acids are situated within the first 30 amino acids of the sequence, supporting that loop 1 and the start of loop 2 of the cardiotoxin structure are the most important for cardiomyocyte toxicity. Using this information, it was proposed as the region of cardiotoxins sequence from which we would make short active synthetic peptides.

Using the mass spectrometry data obtained for the six cardiomyocyte fractions investigated and structure activity relationship (SAR) analysis, three short synthetic peptides were designed:

Peptide 1 - designed using the first 13 amino acids of fraction N.aan_I15_R4 which displayed no toxicity but caused increases to beat rate.

Peptide 2 - designed to contain amino acids important for both high beat rate and high toxicity but with modifications to increase toxicity and increase beat rate

Peptide 3 - designed to have amino acids important for high beat rate and no toxicity, plus and extra lysine for solubility.

The designed peptide sequences were compared to public database sequences using the Basic Local Alignment Search Tool (BLAST) for proteins (BLASTP) from the NCBI (https://blast.ncbi.nim.nih.gov/Blast.cgi). These are unconstrained searches and thus will show hits from all matching organisms.

Peptide 1

BLASTP analysis shows that this sequence is 100% identical to cytotoxin 1 from *Naja annulifera* and *Naja nivea,* with the nearest non cobra sequence having 82% identity to a hypothetical protein from *Solanum commersonii* a wild potato.

Peptide 2

Although peptide 2 is modified, it is 100% identical to 10 *Naja naja* cytotoxins and 3 *Naja kaouthia* sequences. The nearest non-cobra sequence as 84% identity to a Russell's viper (*Daboia russelii*) cytotoxin. The nearest non-snake sequence is only similar to 76% of the sequence with 90% identity of that portion (*Clostridium tarantellae*).

Peptide 3

This peptide has no unique matches in the NCBI databank, with the nearest entry covering 92% of the sequence with 84% identity to *Naja naja* cytotoxin. The nearest non-cobra sequence is from *Clostridia bacterium* with a full match to only 57% of the sequence.

It will be appreciated that these peptides may be modified to improve bioavailability and alter key characteristics to make sure that the compound is selective for rodents and does not persist in the environment. These modifications may include C terminal amides, D amino acids and non-natural amino acids.

Thus, short synthetic peptides have been generated that display functional activity against rodent cardiomyocyte cells. The generation of short, active, synthetic peptides is preferential over the large-scale purification of venom-derived full-length cardiotoxins, as their use will greatly reduce both timescale and cost when upscaled for (commercial) rodenticide production.

All three peptides displayed no toxicity against cardiomyocytes and non-myocyte cell types, good selectivity between rodent and human cardiomyocytes and good thermal stability, as well as an increase in rodent cardiomyocyte beat rate.

The invention is also described in the following numbered paragraphs:

1. A synthetic peptide sequence comprising a sequence based on or derived from Loop I of Elapid or cobra three-finger toxin.

2. The synthetic peptide sequence of paragraph 1, wherein the sequence is based on or derived from amino acid residues 1 to 30 of the three-finger toxin.

3. The synthetic peptide sequence of paragraph 1 or paragraph 2, wherein the sequence has between 10 and 15 amino acid residues, preferably 13 amino acid residues.

4. The synthetic peptide sequence of any one of paragraphs 1 to 3, wherein in the sequence has the following sequence:
LKC(H/N)KL(V/I)PX(V/A)(W/Y)KTC [SEQ ID NO:1]

5. The synthetic peptide sequence of paragraphs 4, wherein the sequence has the sequence of SEQ ID NO:2, SEQ ID NO:3 or SEQ ID NO:4 and variants thereof.

6. The synthetic peptide sequence of any one of paragraphs 1 to 5, wherein the sequence includes one or more hydrophobic amino acids at one or both ends of the sequence.

7. The synthetic peptide sequence of any one of paragraphs 1 to 6, wherein the sequence includes one or more amino acids to enhance solubility in water.

8. The synthetic peptide sequence of any one of paragraphs 1 to 7, wherein the sequence includes one or more synthetic amino acids.

9. The synthetic peptide sequence of any one of paragraphs 1 to 8, wherein the sequence is PEGylated.

10. The synthetic peptide sequence of any one of paragraphs 1 to 9, wherein the sequence includes a detectable tag, preferably a UV-fluorescent tag.

11. The synthetic peptide sequence of any one of paragraphs 1 to 10, wherein the sequence is cardiotoxic, preferably selectively cardiotoxic.

12. The synthetic peptide sequence of any one of paragraphs 1 to 11, wherein the sequence causes a change in heart rate.

13. The synthetic peptide sequence of paragraph 12, wherein the compound is tachycardic and increases heart rate.

14. A rodenticide comprising at least one compound that is cardiotoxic to rodents.

15. The rodenticide of paragraph 14, wherein the compound is selectively cardiotoxic.

16. The rodenticide of paragraph 14 or paragraph 15, wherein the compound has an excitatory cardiotoxic effect.

17. The rodenticide of any one of paragraphs 14 to 16, wherein the compound causes a change in heart rate.

18. The rodenticide of paragraph 17, wherein the compound is tachycardic and increases heart rate.

19. The rodenticide of any one of paragraphs 14 to 18, wherein the compound is an isolated amino acid sequence obtained or derived from snake venom, preferably from a species from the family *Elapidae.*

20. The rodenticide of paragraph 19, wherein the snake venom is derived or obtained from a species of cobra, preferably from the genus *Naja.*

21. The rodenticide of any one of paragraphs 14 to 20, wherein the compound is the compound as claimed in any one of claims 1 to 13.

22. A rodenticide comprising an isolated amino acid sequence derived or obtained from snake venom from a species from the family *Elapidae.*

23. The rodenticide of paragraph 22, wherein the snake venom is derived or obtained from a species of cobra, preferably from a species of the genus *Naja.*

24. The rodenticide of paragraph 22 or paragraph 23, wherein the amino acid sequence is cardiotoxic.

25. The rodenticide of any one of paragraphs 22 to 24, wherein the sequence is selectively cardiotoxic.

26. The rodenticide of paragraph 24 or paragraph 25, wherein the compound has an excitatory cardiotoxic effect.

27. The rodenticide of any one of paragraphs 24 to 26, wherein the compound causes a change in heart beat rate.

28. The rodenticide of paragraph 27, wherein the compound is tachycardic and increases heart beat rate.

29. The rodenticide of any one of paragraphs 22 to 28 wherein the isolated amino acid sequence is a synthetic peptide sequence as claimed in any one of paragraphs 1 to 13.

30. A rodenticide formulation comprising a synthetic peptide sequence as claimed in any one of paragraphs 1 to 13 and a carrier.

31. The rodenticide formulation of paragraph 30, wherein the formulation is formulated as a paste, solid, powder, gel or liquid.

32. The rodenticide formulation of paragraph 30 or paragraph 31, wherein the formulation further includes one or more of: wax (petroleum or plant derived), cereal, grains, nuts, fruit, colourants, flavouring, herbs, spices, essential oils, oil, fat or other material palatable to rodents.

## Claims

1. A rodenticide formulation comprising at least one compound that is selectively cardiotoxic to rodents.

2. The rodenticide formulation of claim 1, wherein the compound has an excitatory cardiotoxic effect.

3. The rodenticide formulation of claim 1 or claim 2, wherein the compound causes a change in heart rate.

4. The rodenticide formulation of claim 3, wherein the compound is tachycardic and increases heart rate.

5. The rodenticide formulation of any one of claims 1 to 4, wherein the compound is an isolated amino acid sequence obtained or derived from snake venom, preferably from a species from the family *Elapidae.*

6. The rodenticide formulation of claim 5, wherein the snake venom is derived or obtained from a species of cobra, preferably from the genus *Naja.*

7. The rodenticide formulation of claim 5 or claim 6, wherein the compound is a synthetic peptide sequence comprising a sequence based on or derived from Loop I of Elapid or cobra three-finger toxin.

8. The rodenticide formulation of claim 7, wherein the sequence is based on or derived from amino acid residues 1 to 30 of the three-finger toxin.

9. The rodenticide formulation of claim 7 or claim 8, wherein the sequence has between 10 and 15 amino acid residues, preferably 13 amino acid residues.

10. The rodenticide formulation of any one of claims 5 to 9, wherein the sequence is:
LKC(H/N)KL(V/I)PX(V/A)(W/Y)KTC [SEQ ID NO:1]

11. The rodenticide formulation of any one of claims 5 to 10, wherein the sequence is SEQ ID NO:2, SEQ ID NO:3 or SEQ ID NO:4 and variants thereof.

12. The rodenticide formulation of any one of claims 1 to 11, wherein the sequence includes:

   a) one or more hydrophobic amino acids at one or both ends of the sequence;
   b) one or more amino acids to enhance solubility in water;
   c) one or more synthetic amino acids; and/or
   d) a detectable tag, preferably a UV-fluorescent tag.

13. The rodenticide formulation of any one of claims 1 to 12, wherein the sequence is PEGylated.

14. The rodenticide formulation of any one of claims 1 to 13, wherein the formulation further includes a carrier.

15. The rodenticide formulation of claim 14, wherein the formulation is formulated as a paste, solid, powder, gel or liquid.

16. The rodenticide formulation of any one of claims 1 to 15, wherein the formulation further includes one or more of: wax (petroleum or plant derived), cereal, grains, nuts, fruit, colourants, flavouring, herbs, spices, essential oils, oil, fat or other material palatable to rodents.

17. Use of a cardiotoxin as a rodenticide, wherein the cardiotoxin is selective for rodents, and wherein the rodenticide is formulated as claimed in any one of claims 1 to 16.

Figure 1A

Cardiac Fibroblast and Cardiomyocyte Toxicty in Response to Fractions at a Dose of 12.5ug/ml

Figure 1B

Cardiac Fibroblast and Cardiomyocyte Toxicty in Response to Fractions at a Dose of 7.5ug/ml

Figure 1C

Figure 2

Percentage of Lysed Erythrocytes Treated with Venom Fractions at a Dose of 7.5 and 0.5ug/ml

Figure 3

% Toxicity in Human IPSC derived Cardiomyocytes after 2hr of Treatment

Figure 4

Thermal Stability Test: % Inhibition of Cardiomyocytes After 24hrs of Treatment with 6ug/ml Fractions Heated at Increasing Temperatures

Figure 5

Figure 6A

Figure 6B

Figure 7A

Toxicity of Synthetic Peptides against Human IPSC Cardiomyocytes

Figure 7B

Toxicity of Synthetic Fractions against Embryonic Chick Cardiomyocytes

Figure 8A

Synthetic Peptide 1

Figure 8B

Synthetic Peptide 2

Figure 8C

Figure 9A

Figure 9B

Thermal Stability of Synthetic Peptide 2

Figure 9C

Thermal Stability of Synthetic Peptide 3

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **BUCKLE** ; **SMITH**. Rodent Pests and their control. Wallingford Oxfordshire, 2015, 330-345
- **VAN DEN BRINK et al.** In Anticoagulant Rodenticides and Wildlife. Springer: Cham, 2018, 1-9
- **JAKOB** ; **BUCKLE**. Emerging Topics in Ecotoxicology (Principles, Approaches and Perspectives). Springer, Cham., 2018, vol. 5
- **MODLINSKA** ; **STRYJEK**. *PLoS ONE*, 2016, vol. 11 (6)
- **NG WY et al.** *Journal of Medical Toxicology*, 2018, vol. 14 (3), 218-228
- **HADLER** ; **BUCKLE**. *Proceedings of the Vert Pest Conference*, 1992, vol. 15, 149-155
- **WATT et al.** *Toxicology Reviews*, 2005, vol. 24 (4), 259-69
- **THIJSSEN**. *Pesticide Sciences*, 1995, vol. 43, 73-78
- **ROST et al.** *Nature*, 2004, vol. 427 (6974), 537-41
- **LI et al.** *Nature*, 2004, vol. 427, 541-544
- **MOONEY et al.** *Nature*, 2018, vol. 8, 4543
- **BUCKLE et al.** *Proceedings of the Sixteenth Vertebrate Pest Conference*, 1994, vol. 7
- **ROST et al.** *BMC genetics*, 2009
- **HORAK et al.** Anticoagulant Rodenticides and Wildlife. Springer: Cham, 2018, 87-108
- **FISHER et al.** *Animals (Basel)*, 2019, vol. 9 (11), 919
- **ERIKSON** ; **URBAN**. A Comparative Approach. *Potential risks of Nine Rodenticides to Birds and Nontarget Mammals*, 2004
- *US Environmental Protection Agency*, 2004
- **EASON et al.** *Ecotoxicology*, 2002, vol. 1, 35-48
- *Environmental Protection Agency*, 2004, www.fluoridealert.org/pesticides/EPA-HQ-OPP-2006-0955-0005.pdf
- **SELJETUN et al.** *Acta Veterinaria Scandinavica*, 2020, vol. 62, 30
- **MEROLA**. *Vet Med*, 2002, vol. 97, 716-722
- **TRAN** ; **KING**. *Journal of Epidemiological Research.*, 2016, vol. 2 (2)
- **MENEZ et al.** *Biochimie*, 1990, vol. 72, 575-588